Europäisches Patentamt

**European Patent Office**

Office européen des brevets

(19)

(11) Publication number: **0 235 312**
**A2**

(12) # EUROPEAN PATENT APPLICATION

(21) Application number: **86101999.0**

(22) Date of filing: **17.02.86**

(51) Int. Cl.⁴: **A 63 B 21/00, A 61 B 5/14, A 61 B 5/22**

(30) Priority: **14.02.86 US 829107**
      **14.02.86 US 829100**

(43) Date of publication of application: **09.09.87**
      **Bulletin 87/37**

(84) Designated Contracting States: **AT BE CH DE FR GB IT LI LU NL SE**

(71) Applicant: **Computerized Sports Equipment, Inc., 1045 Park Avenue, New York New York 10028 (US)**

(72) Inventor: **Silverman,Gordon, 1045 Park Avenue, New York,N.Y.10028 (US)**
      Inventor: **Brudny,Joseph,, 1045 Park Avenue, New York,N.Y.10028 (US)**
      Inventor: **Silverman,Paul,, 34 Maple Avenue,Armonk,, New York 10504 (US)**
      Inventor: **Stundel,Avrom,, 34 Maple Avenue,Armonk, New York 10504 (US)**

(74) Representative: **Wasmeier, Alfons, Dipl.-Ing. et al, Patentanwälte Dipl.-Ing. A.Wasmeier Dipl.-Ing. H.Graf Postfach 382 Greflinger Strasse 7, D-8400 Regensburg 1 (DE)**

(54) **System and method for skill enhancement and behavior modification.**

(57) Structural systems and method are employed including a multiplicity of computers to control the acquisition and enhancement of skilled performance or behavior. Pre-processing computers (8 and 24) each accept signals from any corresponding one of a variety of physiological or other signal transducers either in close proximity to the computer or transmitted to the computer via a telemetering system. The pre-processing computer calculates a number of different measures of this signal and makes the results known to one or more other computers. These other or auxiliary computers control presentation of a display of the data for a human observer, and perform additional calculations which determine if the processed data is within a predetermined range. This range may be a function of time and/or other variables. A measure of the error between the allowed range and the processed data is computed within these auxiliary computers. These results may be used to alter the display. They are also returned to the pre-processing computer where they may alter the pre-processing calculations. Alteration of the display and the pre-processing calculations are adapted to improve skilled performance and behavior without the intervention of a human operator other than the user.

System Block Diagram

# SYSTEM AND METHOD FOR SKILL ENHANCEMENT AND BEHAVIOR MODIFICATION

## Background of the Invention

In the past, the introduction of signals which are electrical analogs of physiological signals into a computer or display has been used primarily for one of three purposes.

(1) Acquisition of data concerning physiological and/or behavioral responses, possibly to some external stimuli.

(2) Physical rehabilitation of human subjects who have lost motor control as a result of some injury or insult to the central nervous system or other parts of the body.

(3) Psychological treatment of altered behavior due to heightened anxiety and stress.

Computer application in the fields of science and medicine led to tremendous advances in these areas. However, these advances have not yet been adapted or reflected in the methodology of instructing people to improve their fitness or performance in sports. In man, the effectors of behavior are primarily the muscles. Their skillful use, in terms of appropriate spatio-temporal and coordinated activity requires learning that leads to eventual patterning or automatic responses. Acquisition of skill is accomplished through very many successful, and properly identified to be successful, repetitive trials. Skill enhancement maximizes the role of the effectors of behavior (muscles) and adds to well-being and fitness.

## Prior Art

There are many skill enhancement machines and systems designed to improve athletic and/or other performance or behavior in healthy, normal individuals. Such presently available systems, however, suffer from a number of limitations. Most do not employ computers for learning, offering only various type of mechanical resistance to cause muscular contractions. The inherent inability of human beings to finely quantify effort without on-line, real-time, augmented feedback makes such contractions either excessive and too widely spread or too inadequate to be effective. The ones that employ computers, use "off-line" techniques for the improvement of skilled behavior. This means that skill improvement occurs as a result of a sequence of operations (knowledge of results). Thus and first, performance or behavioral data is acquired from a subject and stored in a computer. Next, the data is reviewed (after the activity has been completed) and adjustments in the performance are determined. This process is repeated until some behavioral goal is achieved. The delay in the feedback of behavior has been demonstrated to be ineffective for learning or improving skilled performance.

Other machines display a measure of the subject's performance but these may not provide behavioral goals. Thus, a human operator is needed to provide for the target goals. Where the machine includes behavioral goals as part of the display, these goals are often fixed. As a consequence, the machine by itself is incapable of "adaptive" or dynamic operation. This limits the rate and level of performance which can be achieved.

Still other machines do employ some dynamic, adaptive operation. However, these rely on behavioral measures of human performance such as position, and not on underlying physiological signal sources. A subject training signal is provided only when performance falls outside an allowed error range. The subject is unaware of the instantaneous and continuous value of his or her error.

A primary limitation of these machines is related to their organization or architecture. Most machines include a single computer or processor which must carry out all operations

0235312

associated with the acquisition of data, display or results, and calculation of performance goals. Such machines cannot perform all instructions associated with these calculations within a specified time (real time). As a consequence, the subject cannot obtain an accurate representation of his or her behavior. Results are only made available after a considerable delay in time. This limits improvement of skill acquisition.

This criticism of prior art includes typical structures, systems and methods reflected in the following US-Patents:

| J.W. Evans | 3.717.857 | February 20, 1973 |
| J. Brudny | 3.905.355 | September 16, 1975 |
| J.W. James et al | 4.110.918 | September 5, 1978 |
| M.G. Scattergood et al | 4.136.684 | January 3, 1979 |
| S.L. Haas et al | 4.137.566 | January 30, 1979 |
| R.N. Linn | 4.163.941 | August 7, 1979 |
| J.J. Crigler et al | 4.170.225 | October 9, 1979 |
| W.C. Jones et al | 4.250.890 | February 17, 1981 |
| J.I. Cromarty | 4.304.406 | December 8, 1981 |
| E.M. Connolly | 4.337.049 | June 29, 1982 |

**Summary of the Invention**

From one aspect, this invention relates to a novel method which includes a multiplicity of computers. One or more computers are arranged to perform a number of subtasks associated with the acquisition of skilled performance, as might be encountered in a sport or activity such as the improvement of human body condition, tone, or well being. One of the computers is adapted to coordinate the calculations or control functions carried out by the other computers. The sub-tasks include: the transduction and acquisition of any of a variety of physiological signals, calculation of meaningful measure of these electrical analogs, presentation and display (including visual and auditory) of these measures (possibly in the form of a game), comparison of these measures against a criterion which can be simply introduced into one of these computers and which follows an arbitrary function of several parameters including time, and level of performance, and calculation of an error between a desired performance measure and the actual performance measure.

From another aspect, this invention relates to a novel method of skill enhancement, or improvement of well-being which may be incorporated or used in combination with devices of this type such that no further human intervention is needed in order to accomplish the desired performance goals other than the user.

From still another aspect, this invention relates to systems and apparatus for the monitoring and acquisition of signal representations of the physical and/or physiological and/or behavorial, reactions and/or responses and/or movement of a subject person or a bodily part of a subject person, processing such signal representations and providing auditory and/or visual display of the signal representations, on a real time basis for a variety of purposes including without limitation, comparison with a norm, comparison with or maintaining a current criterion, coordination of bodily effort or comparison with one's self, based on physiological response of the person's companion organs.

The novel system and apparatus provides detectors which detect and/or sense body movement and/or function characteristics such as, without limitation, muscular expansion and contraction and, relaxation and tension characteristics, body temperature at selected locations, blood flow and heart function characteristics, using sensing that essentially read the body part function or activity and provide electric signals which represent the body part monitored and include data which define characteristics of the functions or activity being performed and apply the data signals to a unique pre-processor module or sub-computer for pre-processing the data meaningfully. The pre-processor module provides isolators for isolating the subject person from the main power supply, a central processor unit (CPU) programmed to process the data signals for meaningful use by another, associated computer and an interface which provides for compatible coupling of the pre-processor module to the other, associated computer.

In order that the co-computer or pre-processor module be compatably coupled to the associated computer, which may be any one of several off-the-shelf personal computers such as COMMODORE 64 computer or IBM PC personal computer or APPLE II computer, for example, the structure of the interface may differ, depending on the model of the associated computer used since the character of the interface is dictated by the internal and external geometry and design of the associated computer.

The associated computer may include a terminal including a key board with input and output connections, a monitor for temporary visual display, an audio output and, may also include a hard copy printer for providing a permanent display of the data presented on the temporary visual display. The computer may also include provision for accepting the input from a memory or storage device, or ROM, or example which may be in cartridge or disc form such as programmed and used for playing home TV or video games on the monitor or a TV screen.

The subject person or user of the present novel system may be, without limitations, operating or working out on an exercise device or machine which serves as a medium of exercise or skill enhancement, or may be in the process of developing muscular coordination, such as raising one eye brow while maintaining the other eye brow completely in relaxed or rest condition, for example. Detectors or sensors for monitoring the body part desired would be selectively connected to the person so using the system and by watching the screen of the monitor, the person may observe a representation of the action or reaction of the muscular movement so monitored on the screen in meaningful visible traces.

From still a further aspect, the present invention is a system and apparatus for monitoring physiological response of individual companion muscles or organs, for example, left arm and right arm muscles an through the use of electric signals representing the respective muscles and having individual characteristics corresponding to the individual physiological response of the muscles or body organ monitored, play a TV or video game displayed on a TV monitor of the system. Electric signals defining muscular movement and/or reaction may be substituted for electric control signals normally generated by use of a keyboard or joy stick for controlling or moving electronic images around a TV receiver or monitor screen, thus playing a TV or vidoe game. A great advantage of this use of the present invention is to give a child a purpose for exercising or increasing muscular control or coordination without incurring the monotony of repetitious action. This would be effective in taking the child-user's mind off the exercise routine and concentrate on playing the electronic game.

**Objects of the Invention**

It is among the principal objects of this invention to provide improved means for the introduction of physiological data into a computer or electronic game.

Another object of the invention resides in novel means utilized for improving the speed of operation of physiological data processing devices such that calculations on such data can be made available within times which are meaningful for learning of human behavior or response.

A further object of this invention lies in an improved method of introducing into computers variable acquisition and display protocols associated with physiological data processing.

Another object of the invention resides in an improved, novel and automatic method of alternation and adaptation of performance of physiological data processing computers in response to the physiological data.

A further object of this invention is to provide a novel method for development of skill enhancement in conjunction with physiological data processing computers.

Yet another object of this invention is to provide an improved and novel method of skill enhancement which requires no human intervention other than the user and which can accomplish such conditioning of human beings in an automatic manner.

A still further object of this invention is to provide a novel and closed-loop method of learning to carry out repeatedly and appropriately certain behavioral responses, including desired muscular responses.

Another object of this invention is to provide a novel and improved method of narrowing, during the actual performance, and in real time, the gap between simultaneously displayed actual behavioral response and the desired behavioral goal.

A further object of the present invention to provide apparatus for the introduction of physiological data into a computer and display such data in meaningful form on a monitor or TV screen.

A still further object is to provide a system and apparatus that monitors physiological activity or response, converts such activity or response into electric signals corresponding to such activity or response and displays the electric signals in meaningful display on real time basis.

Another object is to provide a system and apparatus which monitors a body part and senses activity and/or response thereof, through picking up bio-electric pulses generated by such activity and provide an electric signal which represents such body part and has characteristics which correspond to the characteristics of the activity or response.

Another object is to provide a system and apparatus for the introduction of physiological data into a multi-stage computer system and display such data in meaningful form in operating or controlling an electric game.

These and other objects will become evident after reading the following disclosure in conjunction with the accompanying drawing.

## Brief Description of the Drawings

Fig. 1   is a diagramatic representation of an embodiment of the system embraced in the present invention;

Fig. 2   is a System Block Diagram of the present invention, illustrating the logical and functional relationships between the transducers, signal conditioning modules, and a plurality of computers;

Fig. 3   is a schematic diagram of the preferred embodiment of the sub-computer or pre-processor module;

Fig. 3a is a schematic diagram of an alternate interface structure for a pre-processor module;

Fig. 4   is a flow diagram of the main loop routine;

Fig. 5   and 5a are flow diagrams of the command processing routine, one diagram being a continuation of the other;

Fig. 5b is a flow diagram of the communication routine;

Fig. 6   is a schematic diagram for a sensor device for monitoring heart rate through blood flow characteristics;

Fig. 7   is a schematic diagram of a sensor device useful for monitoring heart rate using ECG techniques;

Fig. 8   is a diagramatic representation of one form of sensor apparatus usable in practicing the invention;

Fig. 9  is a cross section view of a body exercising device;

Fig. 10 is a schematic diagram of a voltage amplifying
        circuit useful with the invention;

Fig. 11 is a representation of the graphics for an electronic
        game displayed on a TV screen or monitor, useful in
        the system;

Fig. 11a is a representation of alternate graphics for an
        electric game displayed on a TV screen or monitor,
        useful in the system.


**Detailed Description**


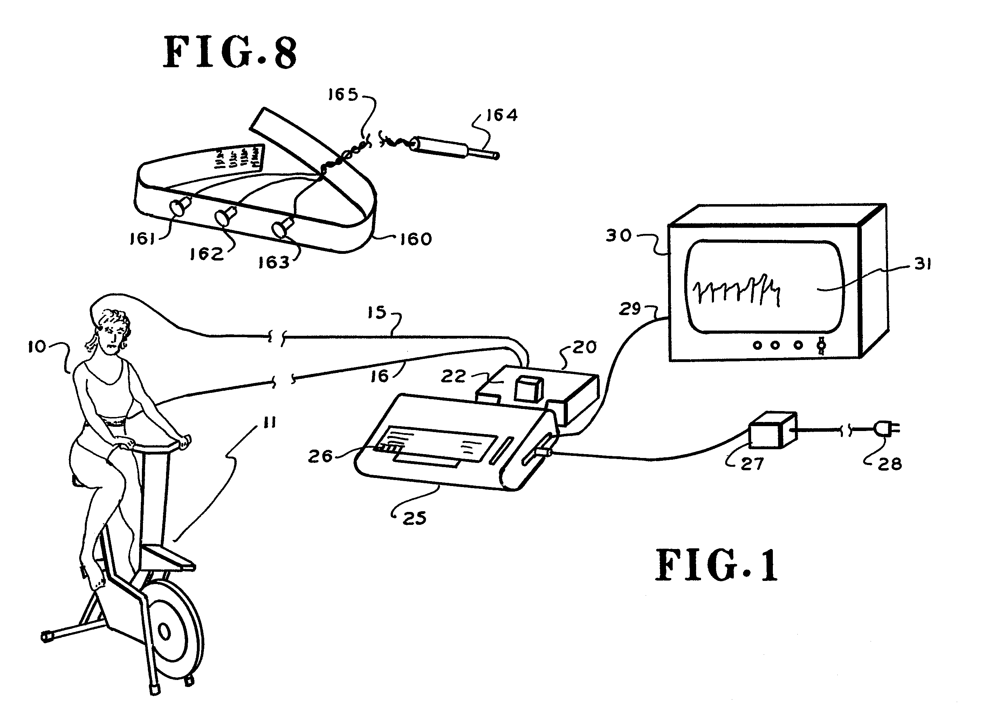

Referring to Fig. 1 a pictorial representation of a person 10
on an exercise machine 11 is shown. The person 10 is in the
process of exercising and has sensors connected to his body.
The sensors are connected to and from part of the system of
the present invention. Although the exercise machine 11 is
illustrated as an exercise bicycle used by the person 10, the
exercise machine represents an exercise machine such as, for
example, a tread mill or rowing machine, or weights or any
other exercise machine or exercise apparatus, in any form,
that a person may use in furtherance of muscular or physical
development, body build-up or tone, muscular coordination
aerobic fitness, and/or skill development. It will also be
appreciated that in some cases, muscular control and/or
coordination may be developed and/or practiced without the
use of apparatus or devices. It will therefore be appreciated
that the present invention may be used by a person whether or
not such person is using an exercise machine or apparatus. In
some cases, as will be discussed below, a person may through
the use of the present invention playing an electronic game
using muscular movement and/or muscular coordination or heart
rate.

Sensors or body function monitors are connected to the person
10 and are coupled to the pre-processor module 20 by leads 15
and 16. Signal conditioning modules may be interposed between
the sensor and the pre-processor module. The sensors typical-
ly monitor physiological reaction such as blood flow and heart beat for example. Lead 15 may be connected to a sensor clipped to the ear of the person for monitoring blood flow through the flesh of the ear while lead 16 may be connected to a sensor securred to the chest for monitoring heart activity.

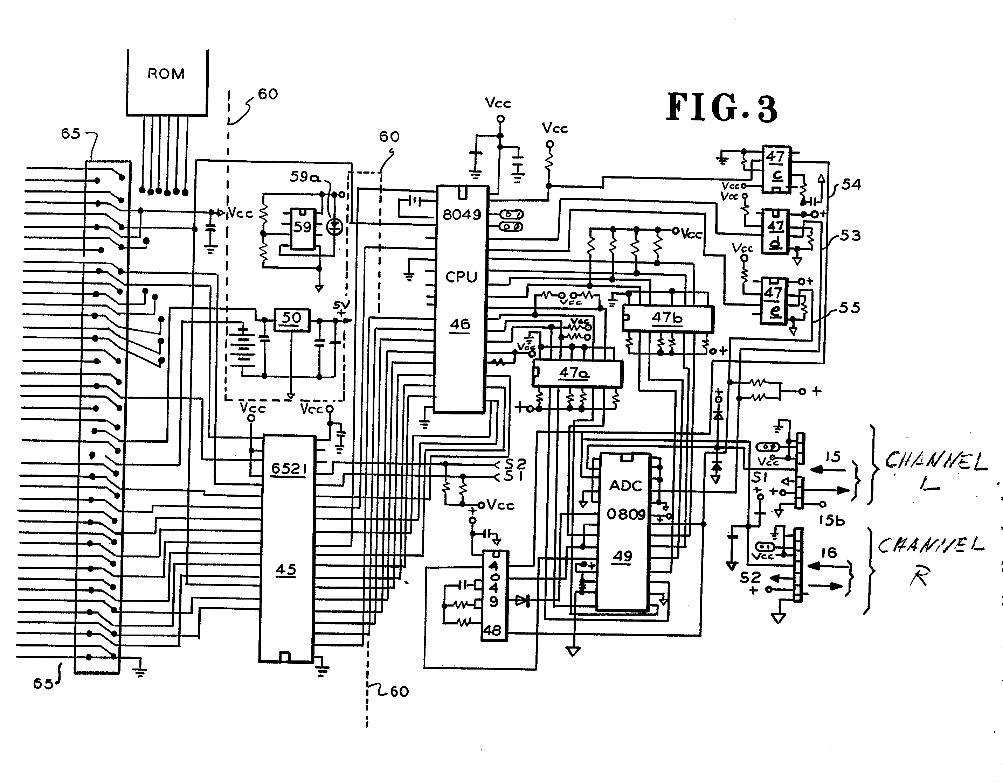

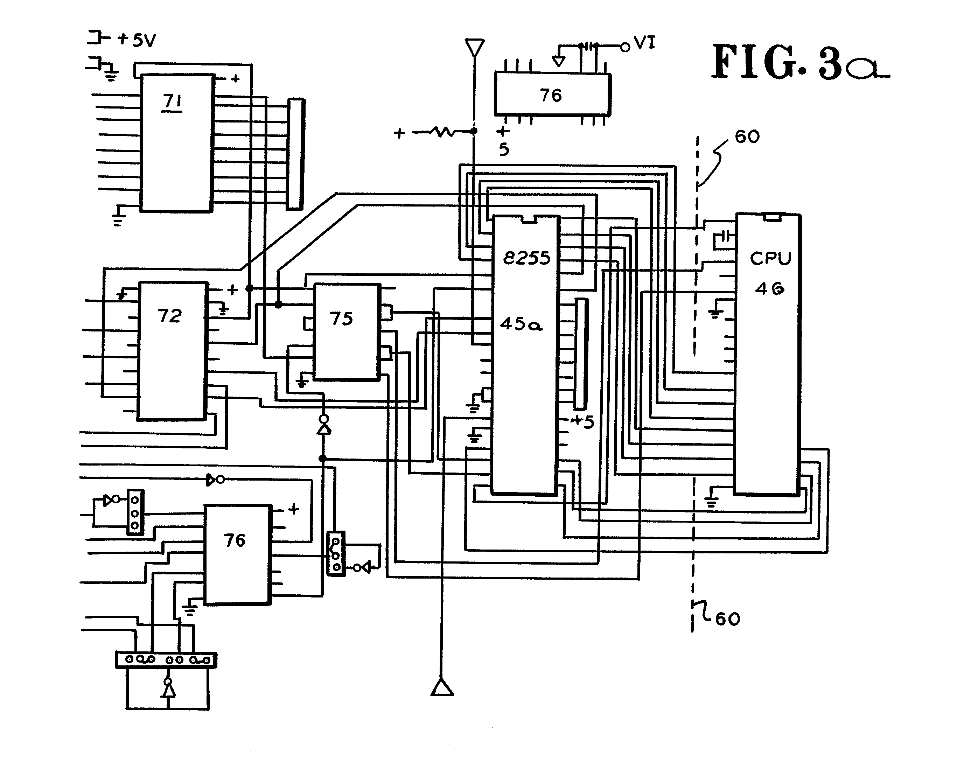

The pre-processor module 20 is illustrated as being connected to or plugged into a computer 25. The computer 25 may be in the form of a personal computer, such as an off-the-shelf COMMODORE 64 Computer, marketed by Commodore Business Machines, Inc., for example. The pre-processor module 20 is illustrated as having inserted therein a cartridge 22, which represents a programmable memory device, which may be programmed and used for playing electronic games. As arranged, as more completely shown in Fig. 3, a conventional electronic game cartridge may be inserted into the pre-processor module while the module is inserted into the computer and one may play the electric game using the computer, without removing the pre-processor module from the computer and inserting the cartridge 22 into the computer, as in normal practice.

The cartridge 22, or ROM, may include a programmable device programmed to provide other graphics or animation including moving and/or movable characters and/or images and the characters and/or images may be controlled and/or manipulate by signals representing characteristics of physiological response or behavior in coordination with the computer.

The computer 25 includes a keyboard 26 and a remote power converter 27, which is plugged into the computer. The power converter also includes a normal house current recepeticle plug 28 for connection to a normal house current supply. The system also includes a monitor 30 which is illustrated as connected to the computer terminal 25 via lead 29. The monitor 30 includes a screen 31 which may be used to display representations of the characteristics of the physiological reactions sensed by the sensor attached to the person 10 or other display as discussed.

The computer may also include a hard copy printer (not illustrated) and/or a temporary memory or storage device for temporarily storing data (not shown).

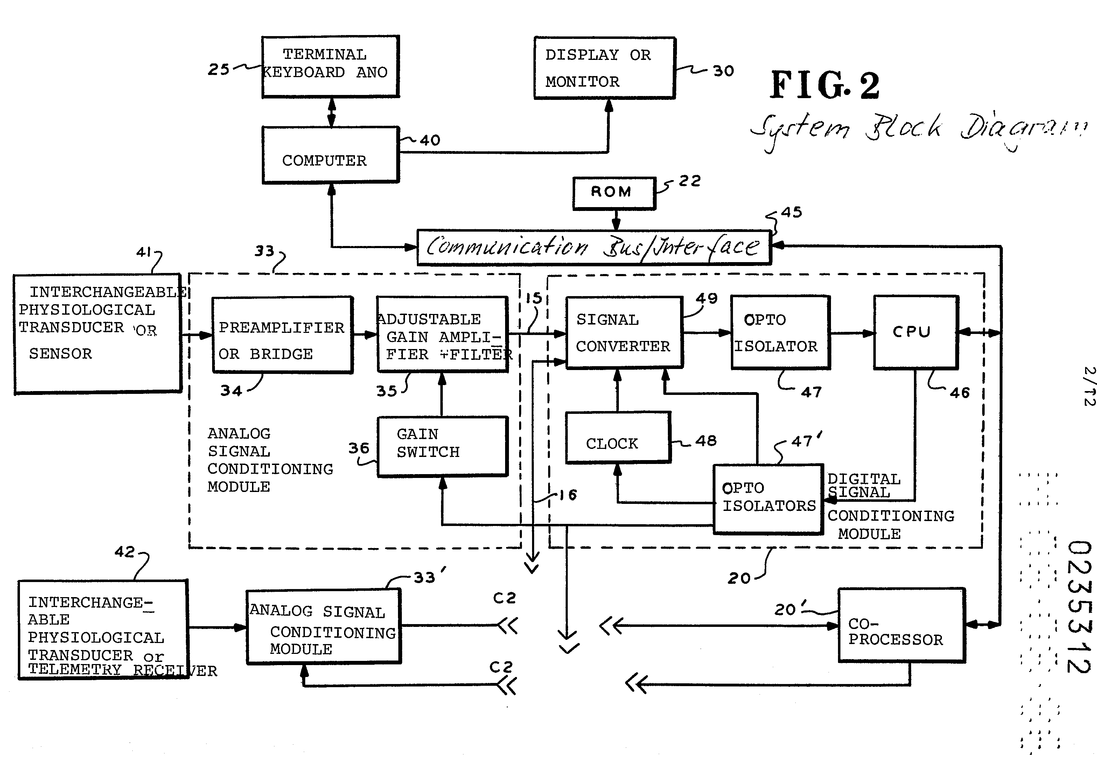

One embodiment of the present invention is shown in the System Block Diagram of Fig. 2. The skill enhancement system is divided into a fixed portion and a system of variable or replaceable elements. The fixed portion is intended to meet all possible physiological and/or behavioral environments and applications. The invention can be adapted, without modification, to industrial or scientific purposes, providing cost effective instrumental solutions to data acquisition applications in these environments. Binary coded sequences stored in both the fixed and replaceable program memory means found in the present invention provide for control of the system. In addition, signal transducers convert physiological, behavioral, or other sources of information into electrical equivalents. Binary sequences stored within the various memory means, control displays such as those which may be found in electronic games, and also control the signal processing that is to be performed on the electrical signals received from the signal transducers.

A number of different sensors or transducers can be directly integrated into the system. The following, but not exhaustive list, indicates a number of transducers which are compatible with the skill enhancement system together with their appropriete physiological source. These transducers form part of the replaceable portion of the skill enhancement system.

| Physiological Function | Transducer |
|---|---|
| Muscle (EMG) | Electrodes, piezoelectric |
| Brain Wave (EEG) | Electrodes |
| Heart rate, plose rate and blood pressure | Photo detection |
| Respiration | Resistance (strain gauge) |
| Temperature | Resistance (thermistor) |
| Position | Resistance (goniometer) |

| Force | Resistance (spring deflection) |
| Galvanic skin resistance | Resistance |

Signals from these transducers may be connected or integrated directly into the system. In addition, transducer signals may be used to modulate a carrier signal. The resultant signal can be transmitted from a remote location to the skill enhancement system where a receiver carries out demodulation. The resultant demodulated information, physiological or other, can be integrated into the present invention.

Any or all of a plurality of interchangeable physiological transducers or telemetry receivers 41 and 42 generate signals or data in correspondence with human electrical activity, changes in body volumes, temperature, or positional changes of limbs or other parts of the human anatomy. These signals are transmitted to a corresponding signal conditioning module as 33 and 33' adapted to receive such signals and to process the data such that it is suitable for transmission to a sub-computer for further processing. In order to carry out such processing an analog signal conditioning module 33 accepts signals from interchangeable physiological transducer or telemetry receiver 41 or 42 and transmits these to preamplifier or bridge 34. If, for example, the interchangeable physiological transducer or sensor 41 includes resistive transduction, its effect is detected in a standard bridge circuit (in preamplifier or bridge 34) well known to those familiar with the art. If interchangeable physiological transducer or sensor 41 generates or detects signals which are electrical in nature, such as those of muscle (EMG), brain waves (EEG), heart (EKG) or force (piezoelectric), then such signals are amplified by preamplifier or bridge 34. Outputs of preamplifier or bridge 34 are further amplified in adjustable gain amplifier and filter 35. Gain switch determines the amplification factor or gain associated with adjustable gain amplifier and filter 35. Operation of gain switch 36 will be discussed below: However, the following description of the need for adjustable gain amplifier and filter 35 in the present invention is deemed opposite. When
the skill enhancement system is used in applications requiring measurement of heart rate, the amplifier gain will be set
to a high value. The reason for this is linked to the
transduction mechanism for detection of heart rate. In the
example of heart rate detection (as part of a method of
behavior modification) an optical source may illuminate a
user's ear, or other part of the human anatomy. The user's
blood flow which is related to the occurrence of a heart
modulates the light received at an optical detector located
near the source of illumination. This modulated signal
eventually appears at the input to the analog signal conditioning module 33. In order to computer heart rate, only the
time between beats is important. Consequently, only the time
at which a modulation peak occurs is of interest. The
amplifier gain of adjustable gain amplifier and filter 35 is
set at a high value by the combination of gain switch 36 and
CPU 46 in order to detect such modulation peaks.

In the example of the measurement of muscle responses in
which EMG measurement is appropriate, the gain or amplification factor of adjustable gain amplifier and filter 35 by the
combination of CPU or computer 46 and gain switch 36 will be
adjusted in a different manner. If the amplification is too
high, the system will "saturate" and any measures of such EMG
activity will go "off scale", especially when a user makes a
muscular contraction. The gain must be adjusted so that this
does not happen. Thus, the gain setting must be flexible and
vary from one user to the next. The gain can be adjusted such
that maximum measurements from a given user does not saturate
the system. Signals are "normalized" with respect to such a
user. Any methodology subsequently employed to modify
behavior is made independent of absolute signal level - the
system will work for "weak" and "strong" users alike.

The amplified and filtered signals from the analog signal
conditioning module 33 are electrical analog signals which
are equivalents of physiological data. These signals are
transmitted via lead 15 (or 16) to the digital signal conditioning module 20. The analog signals are then converted to digital signals which preserve the integrity of the analog signals. This is accomplished by the signal converter 49. These converted digital signals are transmitted to CPU 46 via opto-isolator 47. Opto-isolator 47 is included in order to prevent or minimize electrical shock hazard to a user. Any and all circuits of the skill enhancement system which are in contact with a user are powered by batteries. These circuits have no direct contact with the power mains. The equipotential pluses which are generated by the signal converter 49 pass through opto-isolator 47 to components which may have contact with the power mains.

The signals reaching CPU 46, which forms part of the fixed portion of the skill enhancement system, are digital signal having characteristics which correspond to characteristics of human physiological or other data detected. The frequency variation contains the information associated with the original sources. CPU 46 contains a plurality of binary sequences. A multiplicity of these sequences are adapted to process the train of digital pulses received from opto-isolator 47. The table shown below indicates what processing calculations the multiplicity of binary sequences within computer 46 are adapted to perform, and under what circumstances such calculations would be relevant. Co-processor 20' performs similar functions on signals received analog signal conditioning module 33'.

It will be noted, in respect of Fig. 3, that in the preferred form of the invention the sub-computer or pre-processor module 20 includes two inputs or channels. Interchangeable physiological transducer or sensor 41 may be monitoring one body function while interchangeable physiological transducer or telemetry receiver 42 may be monitoring a different body function and both signals representing the bodily functions may be applied to the same sub-computer, or pre-processor module such through channel 1 (lead 15) and channel 2 (lead 16), respectively. This is represented at the interchange of connections C2 and C2'. Alternatively a second sub-computer or pre-processor module 20' may be used, if desired.

As set forth below the example of a computer program for CPU, 46 considers the use of a two channel system although other programs may be written.

It will be appreciated other programs may be written which consider only one channel or more than two channels. This alteration from the preferred embodiment may be desired in light of the table below.

## TABLE OF BUILD-IN DATA PROCESSING CALCULATIONS

| Computation | Mathematical Function | Uses |
|---|---|---|
| Count number of pulses per unit time. | Integration | Muscle activity Brain activity |
| Difference between highest and lowest frequency. | Peak to peak detection | Respiration |
| Time of occurrence of highest frequency. | Peak detection | Heart rate |
| Frequency at any (all) point(s) in time. | Signal magnitude (analog to digital conversion) | Temperature Limb position |

This table is not exhaustive but is representative of the possible uses. The calculation performed on the data are functions of the program written into the microprocessor or the CPU. These calculations are performed in cyclic appearance under the control of the clock 48. The computer 40, which may be a host computer, makes requests of the CPU 46 for data and the requested data is provided over the interface which includes a communication chip 45 (Fig. 3). Thus, the host computer 40 transmits commands for data or parameters to such computer 20 or 20'. These sub-computers may return or transmit computed data or results to the host computer 40. The processed information which is sent from pre-processor module 20 and/or 20´ to computer 40 can subsequently be incorporated into a display or electronic game. This is accomplished by computer 40 which contains binary coded sequences within its memory for control of a display 30. These binary coded sequenes are adapted to accept such processed data and to alter or modify the display 11 which may be associated with an electronic game. Such display 11 can be either visual or auditory or tactile, or a combination of being visual, auditory and tactile.

Such a display provides a user with a knowledge of his or her physiological or motor behavior or performance. The combination of interchangeable physiological transducer or sensor 41 (or 42), signal conditioning module 33 (and/or 33´), pre-processor or module 20, (and/or 20´), host computer 40, display 30, and terminal 25 can be incorporated into a method of training athletic performance, including sports and fitness. Such a method can be based on comparison of quantified information reflecting actual and desired and/or optimal activity in key muscles or other parts of the human physiology involved in the production of a particular behavior or skilled performance.

The force and velocity of varying muscular activity can be determined in "real time" (in which -results are provided to a subject in time to alter behavior or performance during its envolvement and not in ordinately delayed from the time of occurance of a behavior) by the present invention and displayed for a user. The user can integrate such knowledge of performance during activity and compare results with ideal or desired performance levels which can also be integrated into the display or electronic game. Such ideal and/or optimal performance goals can be based upon or normalized with respect to an individual´s present level of performance. Alteration of the user´s performance towards a more desirable value of force and velocity of muscular activity is achieved in consecutive trials or behaviors by reducing the gap between the actual and the desired performance as reflected in the display or electronic game. This training procedure takes place in a step-by-step manner, with the user learning
the specific control over spatial temporal activity of key
muscles or other parts of the human anatomy or physiology
until coordinated control is achieved and patterning or an
automatic, dynamic, skilled behavior or movement is established. At this stage there is no further need for the
cognitive information provided during training by the closed
loop interaction specific to the described method.

In order to provide control of the entire method or sequence
of operation, the user may communicate intentions such as
"begin operation" terminal 25 which is connected to computer
40. Terminal 25 comprises a keyboard and is well known to
those familiar with the art. Computer 40 receives binary
sequences which are coded representations of alphanumeric
characters from the keyboard of terminal 25 and transmits
binary coded sequences representative of alphanumeric
characters to the monitor 30.

Fig. 3 represents, in schematic form, a preferred embodiment
of the digital signal conditioning or pre-processor module
structured so that the module is compatable for use with a
COMMODORE 64 Computer. A broken line 60 partitions the
circuit, indicating that the partitioned part or section, the
computer/interface section, may be removed and another
computer/interface section may be substituted therefore.

Fig. 3a represents, in schematic form an alternate computer-
/interface section which may be substituted for the computer-
/interface section in Fig. 3. This structure of interface
makes the pre-processor module compatable with the input/-
output lines of the IBM PC Personal Computer.

Referring to Fig. 3, the schematic structure 65 represents
the connections to the computer 40 to which the pre-processor
module or digital signal conditioning module would be
connected. The lead Vc represents a voltage output from the
computer applied to simularly marked terminals throughout the
module. Block 45 represents the communication chip of the interface, which may be an off-the-shelf chip 6521 marketed by Hatachi. The communication chip serves to connect the CPU, Block 46 to the computer 40. The central processing unit (CPU) of the digital signal conditioning module may be an off-the-shelf microprocessor chip, such as the 8049, marketed by Intel Corporation. The CPU is connected to the isolators 47a, 47b, 47c, 47d and 47e, which preferably are optical isolators, isolators 47a and 47b being quad isolators, each having four isolated lines and isolators 47c, 47d and 47e each being single line optic isolators. The isolators 47a and 47b pass data information or signals and the isolators 47c, 47d and 47e pass control data or signals. Isolators 47d handles address control, via lead 53, calling for channel 1 or channel 2, as dictated by the CPU. Although only two channels are represented in this schematic diagram obviously additional channels may be used if desired. Additional channels may require minor changes in circuit structure, which will be familiar to those skilled to the art. Isolator 47c controls the end of the conversion by the analog to digital converter (ADC), block 49 through lead 54. Isolator 47e controls the start of the conversion via lead 55.

One chip that may be used to provide the clock function is a chip marketed by RCA, 4049, represented by block 48.

The analog to digital conversion function represented by block 49 may be accomplished using on off-the-shelf chip marketed by National Semi-Conductor Corporation ADC0609. This component selects signals from channel 1 or channel 2, upon command of the CPU via isolator 47d, converts the analog input signals to digital data signals having characteristics which correspond to characteristics of the analog input signal from which the data signal was converted. The leads 15 and 16 represent the leads of channel 1 and channel 2 respectively going to the interchangeable physiological transducer or sensors 41 or 42. The terminals S1 and S2 at the channcels 1 and 2 respectively are connected to the terminals S1 and S2 on the communication chip 45 and may be used for mechanically induced signals, such as a button or a

19    0235312

joy-stick, for example. The terminals 00 and 01 of channels 1 and 2 may be connected, via isolation components, to correspondingly identified terminals on block 46, CPU. These terminals may be used to provide a gain control output between the CPU and the analog signal conditioning module. In such event it would be preferred to pass the signal through an isolator. If no gain control signal is required, these terminals may be used to provide control over a remote alarm or indicator such as a lamp or buzzer, not shown.

The block 50 represents the regulated supply which is driven by a battery, such as a 9 volt DC and provides a regulated 5 volt DC output represented by a plus sign (+). The regulated supply is applied to terminals in the isolated section of the pre-processor module indicated by a plus sign (+).

The block 59 represents a low battery indicator. It is connected to the 9 volt battery and when the 9 volt battery runs low the LED 59a becomes illuminated.

Referring to Fig. 3a, when a digital signal procesor module, such as 20, for example is used in conjunction with an IBM PC Computer, the geometry of the computer is such that the components of the pre-processor module may be structured on to a circuit board that is inserted into the computer.

The manufacturer makes available for purchase as an off-the-shelf component, a circuit board on which is already provided some components and structure which provide for compatably coupling peripheral apparatus to the computer. The schematic diagram in Fig. 3a shows this alternate interface structured with two buffers 71 and 72 and two address decoders 75 and 76. This structure is compatable for use with a communication chip 45a such as the 8244 marketed by Intel Corporation. This structured interface is dictated by the structure of the IBM PC Computer, the communication chip 8255 being connected to the CPU 46 (across broken line 60), the reminder of the pre-processor module or digital signal conditioning module being identical to that shown in Fig. 3 except that a separate regulated 5 volt DC supply is not required since a 5 volt DC supply is available from the IBM PC Computer. In addition, a 9 volt battery indicator need not be used as in the structure shown in Fig. 3. In order to ensure that an isolated of the 5 volt supply is available, a DC to DC converter block 76 is provided which supplies an isolated 5 volt DC to the isolated components of the pre-processor module having this alternate computer/interface structure as well as to the person of the user.

The structure and geometry of the IBM PC Computer is designed to use a programmable storage device in disc form. This may require a disc drive as peripheral equipment for the IBM PC Computer.

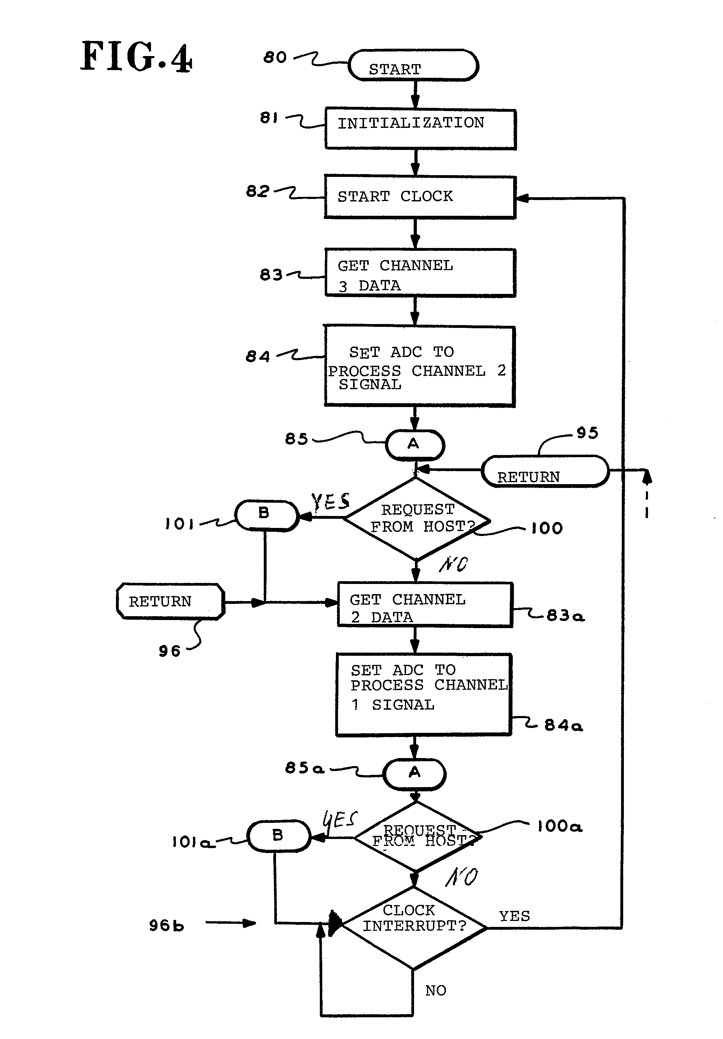

Fig. 4 is a flow diagram generally describing the main loop routine of the program set forth below. At power turn-on or start 80, the pre-processor or CPU 46, is automatically driven to initiatization 81, which represents a start position. The CPU is driven to start position from any position or location it was last in, in respect of the program, when the system was last turned off or shut down. The start clock 82 functions to start the internal clock in the CPU to time the cyclic routine. It will be appreciated by those skilled in the art, that the conversion process, from analog to digital, by the ADC and the transmission of data by the isolators between the ADC and the CPU takes time due to the nature of the components involved. The structure of the software program tries to take this time factor into account by calling for the performance of one operation while waiting for the other operation to occur. Thus, the routine calls for, get channel 1 data, 83 and set ADC to convert channel 2 signal 84, and thus sets up, for example, conversion of channel 2 signals from analog form to digital form while performing calculations on or processing data of channel 2 in accordance with commands last received from the computer.

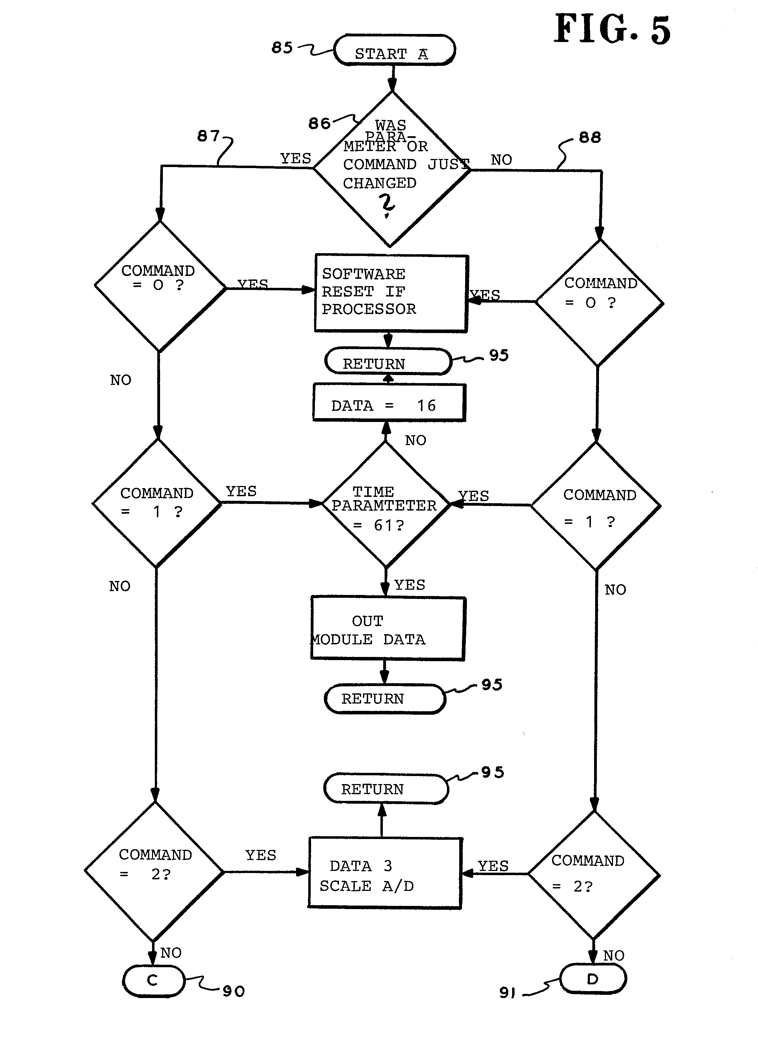

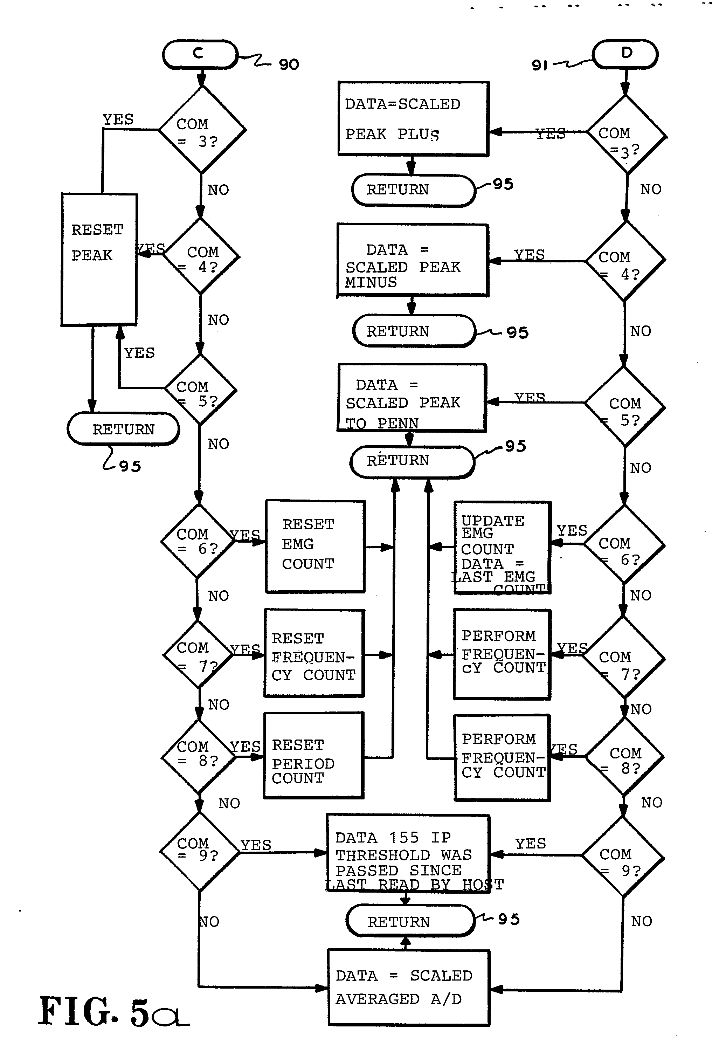

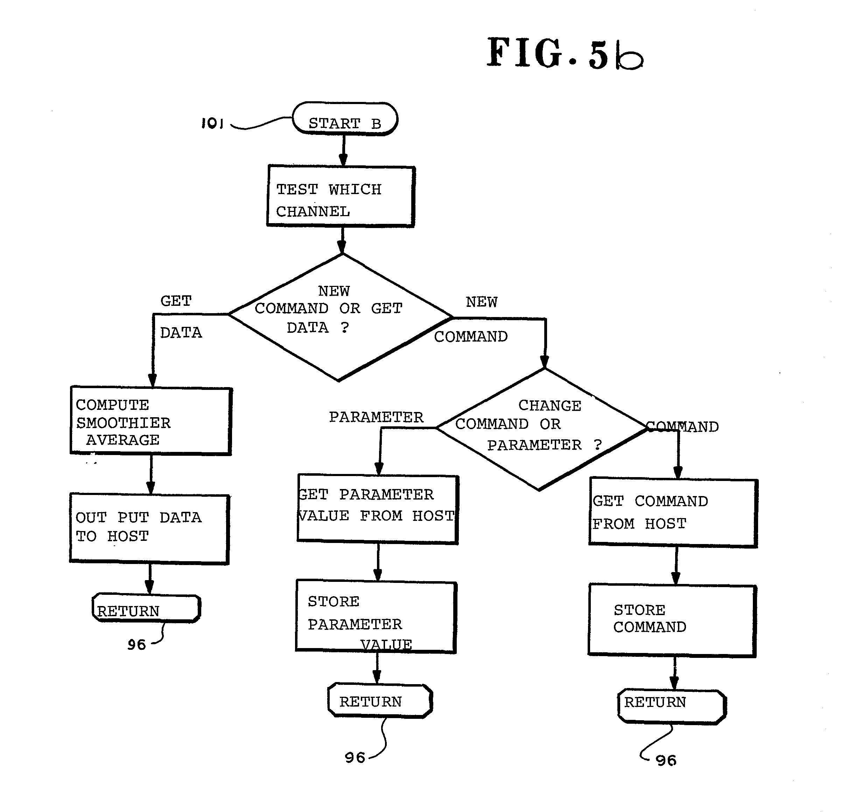

The command processing routine, such as set forth in Fig. 5
and 5a, starts at step or position A, 85 where commands
related to channel 1 data are processed. During this time
channel 2 signals are converted. The command routine determines whether or not the parameter or command was just
changed, 86 and if so (yes 87), (no 88), what the current
command is. Return is made to RETURN 95 (Fig. 4) to seek
another request of the host 100. If there is no request the
routine follows to obtain channel 2 data, 83a and set ADC to
convert channel 1 signals 64a if there is a request from the
host 100 the routine follows to B 101 going into the communi-
catin routine (Fig. 5b) to determine the channel number of
the data requested and the process to be performed or the
requested handling of the data. Return from the communication
routine is made to position 96 where the routine continuous
at step 83a.

It is well known that when a person exercises the rate of
heart activity increases thereby increasing the blood flow
through the body. Thus, changes in blood flow may be used as
a factor in detecting heart activity.

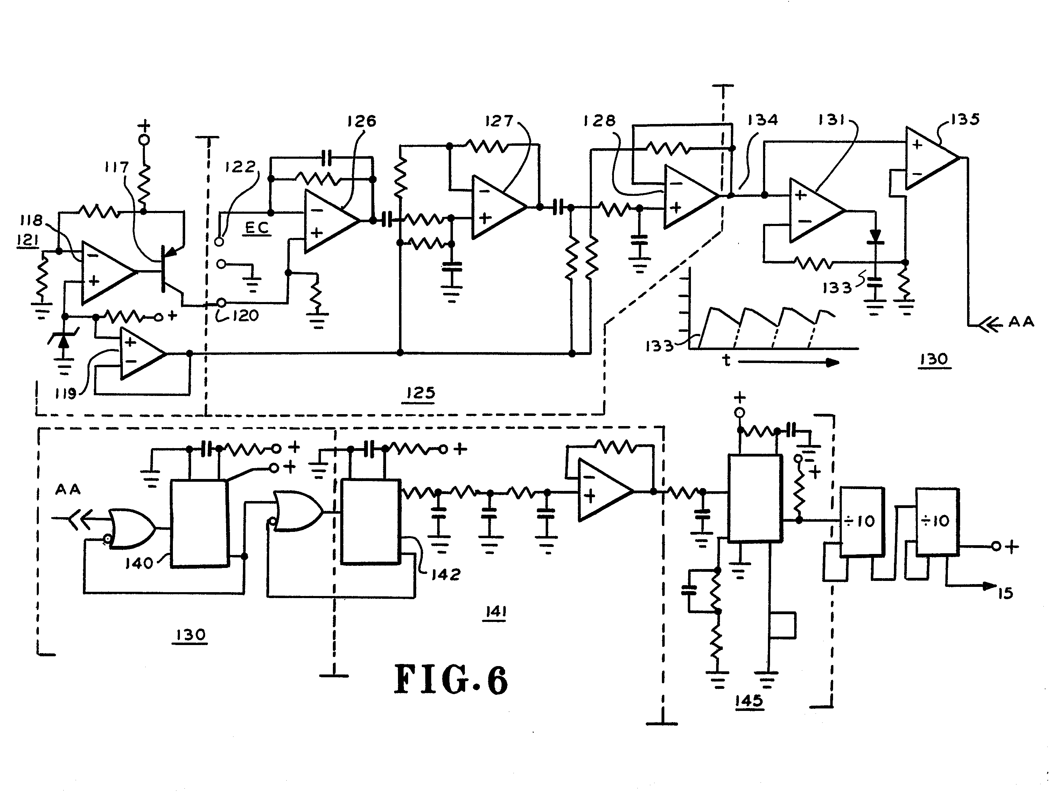

Fig. 6 is a schematic circuit diagram of an optical blood
sensor and amplification circuit for providing data on heart
activity from blood flow. Preferably, an optical sensor
element, in the form of an ear clip EC, is attached to the
ear with a light source, such as a light emitting diode,
(LED) 120 on one side of the ear and a photodetector 122 on
the other side of the same ear positioned to received light
from the LED through the flesh of the ear. The intensity of
light passing through th flesh of the ear changes as a
function of the volume of blood flowing through the flesh.
Since the electric output of the photodetector 122 is a
function of the intensity of light received from the LED 120,
then the change in electric output of the photodetector is a
function of the change in blood flow. The LED 120 is driven
by a constant current circuit 121 including amplifier 118
which drives translator 117. Amplifier 119 provides a
constant reference applied to the amplifiers 127 and 128. The output of the photodetector 121 is applied to a triple stage amplifier circuit 125 including amplifiers 126, 127 and 128. The amplified output, a pulse at 134 is applied to a peak detector 131 which provides a peak pulse at 133. The R-C network filters the signals and permits decay, with time (t) so that the next pulse at 134 may be detected and a peak pulse at 133 provided even though the maximum amplitude of the pulses at 133 is substantially constant. A comparitor 135 compares the pulse 134 with peak pulse 133 and provides a trigger pulse for triggering one shot multivibrator 140. The connectors AA are joined to continue the circuit. The multivibrator 140 has an upper frequency level of 300 shots per minute since the output of this one shot is not intended to exceed heart beat frequency. The output of the one shot multivibrator 140 is applied to another one shot multivibrator 142 which is part of a frequency to voltage convert 141. A voltage to frequency converter 145 provides a frequency output at a multiplied factor which is then divided by one hundred using two divide-by-ten dividers, 147 and 148 to provide an output at 15. The output 15 is an analog voltage, the frequency of which is a function of the incremental time between changes in blood flow through the ear.

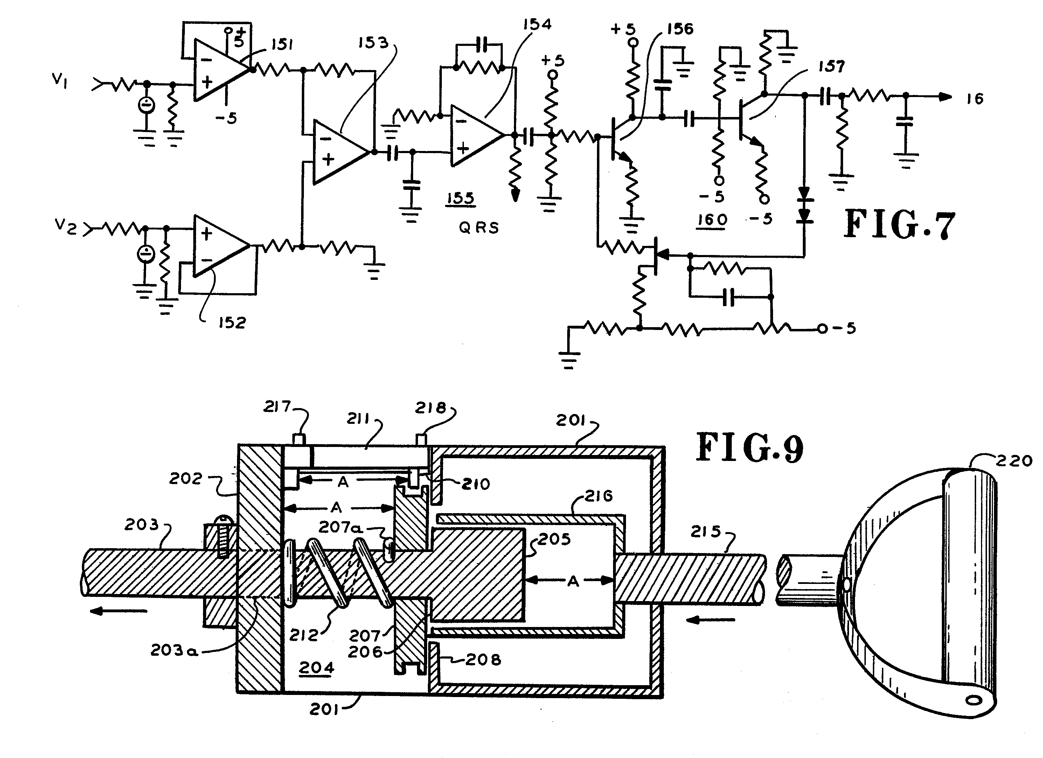

Fig. 7 is a schematic diagram of a circuit that may be used along with ECG (electrocardiogram) techniques for obtaining heart action data. As known in ECG techniques, each of at least two heart sensing electrodes V1 and V2 may be selectively coupled to the chest of a person for sensing cardioelectric response. The electric impulses may be individually amplified using instrumentation amplification techniques such as the structure formed by amplifiers 151, 152, and 153. The signals may be further amplified and filtered by amplifier 154, the output of which appears at terminal 155 as QRS, the raw heart beat signal. This complex signal is further filtered and refined by applying the signal to the transistor network 156 and 157 with automatic gain control 160. This provides a substantially clean analog pulse which corresponds to the heart beat as opposed to the complex wave QRS. The output pulse signal may be applied via lead 16, for example, to the input channel of the pre-processor module.

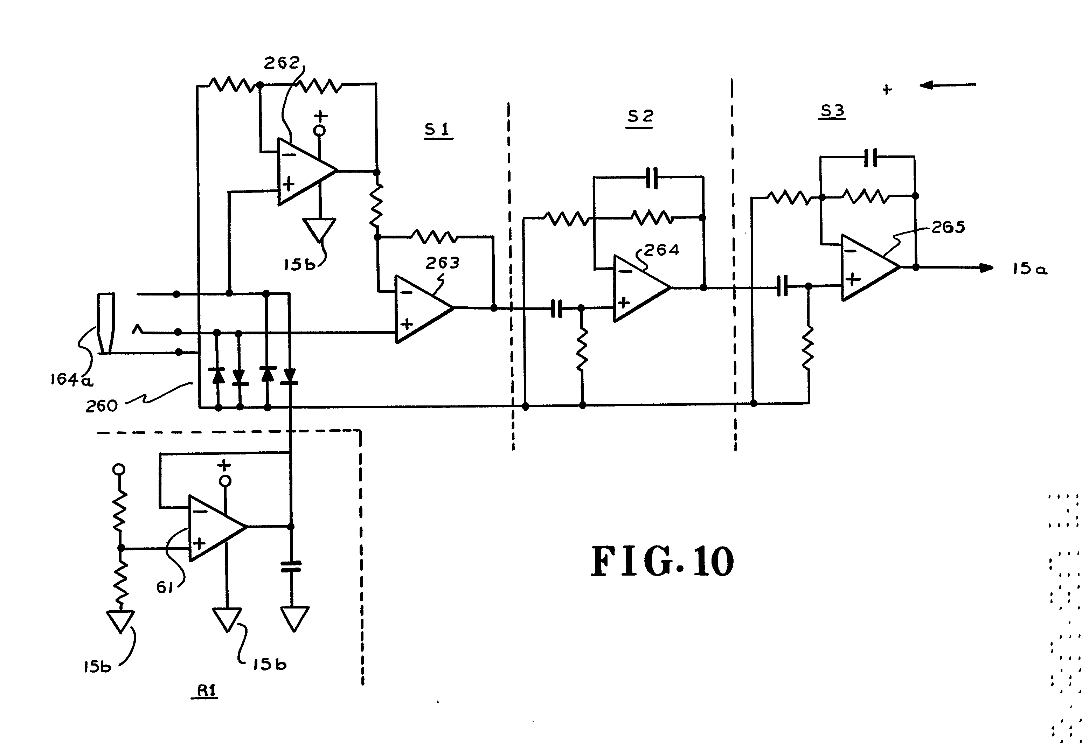

Fig. 8 illustrates one form of transducer or sensor arrangement for detecting and monitoring physiological reaction in the muscles of the forehead, for example. Three sensor elements 161, 162 and 163 are mounted in a headband 160. The headband 160 is secured about the head so that the elements make contact with the forehead for monitoring the electric pulses generated by muscular action. When muscles so monitored are expanded and/or contacted electric pulses are generated within the muscle and these pulses are sensed by the sensor elements and are transmitted via leads 165 to the male component 164 of a jack-plug connector. The male component 164 may be plugged into a female component 164a shown in Fig. 10, for example, where the signals from the sensor elements may be amplified. The transducer arrangement in Fig. 8 may be represented in Fig. 2 by the block 41 and 42 and the impulses from the sensing elements may be applied to the analog signal conditioning module 33 or 33' such as represented in Fig. 2.

Although the present system and the apparatus used therein is directed toward providing instantanious data on physiological reaction of the individual practicing the invention, the system has other potential uses. One such alternate use is the determination of the relationship of a selected physiological reaction or effort relative to the amount of work or effort applied to a device, on real time basis. Sensors may be used to monitor and provide electric data on physiological reaction of a person, as described above, on one channel and the second or another channel may be used as an input for providing data on the work applied to the exercise device from the exercise device itself. One such value may be the speed at which a person pedals an exercise bicycle or the elevation and/or speed of a treadmill or the force overcome by lifting, pulling or pushing, for example.

One novel exercise device that may be used to provide work or exercise data on a real time basis is represented in Fig. 9, in cross section view. The exercise device includes a frame or housing 201 with the end member 202. The end member 202 has a hole 202a which permits shaft 203 to pass into a chamber 204. Shaft 203 had an enlarged section 205 forming a shoulder 206 forming the other end of the chamber 204. A flange 207 has a hole therein and shaft 203 passes through the hole 207a. The flange 207 is grooved about its periphery and holds captive a movable member 210 of a potentiometer 211 mounted in the wall of the housing 201. A spring 212, positioned about the shaft 203 and between the end member 202 and flange 207 provides compression resistance when the flange 207 is moved by pulling the shaft 203 so that the shoulder 206 of the enlarged portion 205 of shaft 203 impinges against the flange forcing the flange 207 toward the end member 202 against the pressure of the spring 212. The chamber 204 has a usable depth "A" which is substantially equal to the travel of the moving member 210 of the potentiometer 211. As is well known a potentiometer includes a resistance (not shown) which may be measured electrically. The resistance is connected to terminal 217, at one end to the moving member 210 at the other end and when there is a distance "A" between the stop at 217 and the moving member 210 the electrical resistance is maximum. As the flange 207 is moved into the chamber space 204 thereby reducing the distance "A" the movable member 210 is carried in the groove of the flange thereby reducing the distance "A" between the end electric terminal 217 and the movable member 210 and thus the resistance in potentiometer 211. The change in electrical resistance in the potentiometer is then a function of the force of the spring which has been overcome by the physical effort applied to the exercise device. The terminal 218 may be connected electrically to the moving member 210 and the potentiometer may be used as a component in an electric circuit which will defect the change in resistance in the potentiometer.

The present novel exercise device may be used to measure a pulling force or a pushing force. When used to measure a pulling force, the handle or grip (not shown) on the shaft 203 is pulled while the housing 201 is secured to obtain the internal action described above.

When used to measure a pushing force, a force may be applied via grip 220 to shaft 215 which is connected to the open cup member 216. The open end member 216 impinges against the flange 207 forcing flange 207 against the spring pressure and carrying the moving members 210 of the potentiometer along as the distance "A" is decreased by the travel of the pushed flange.

It is well known to use devices for detecting electro-biologic impulses. Such devices may be used as elements in the sensor device shown in Fig. 8, however, it is also known that the outputs of such elements are extremely small with respect to power levels and may require amplification in order to be effectively used. Thus, in interests of complete disclosure Fig. 10 is presented which shows a schematic diagram of a useful amplification circuit. The male component 164 in Fig. 8, of a jack-plug connector may be inserted into the female component 164a shown in Fig. 10, thereby feeding electric signals picked up by the sensor elements into the amplifier. The diode network 260 provides protection against voltage serges which, if not shunted out would destroy the amplifier. The terminals with a plus (+) receive the regulated 5 volt DC supply for driving the amplifier circuit. The circuit return is connected via 15b to the return terminal 15b of the pre-processor module. Amplifier 261 in section R1 establishes a reference voltage for the amplification circuit. Stage S1 including amplifier 262 and 263 is a double stage differential input amplifier, with gain. The first stage amplified signal is applied to the second stage S2 which is a single ended input amplifier with gain and filtering. Amplifier 264 is provided with feedback filtering. The signal out of amplifier 264 is applied to the third stage S3 which includes amplifier 265 which is another single ended amplifier stage

26

0235312

with gain and feedback filtering. The output signal at 15a would be applied to the terminal 15 as an input to the pre-processor module.

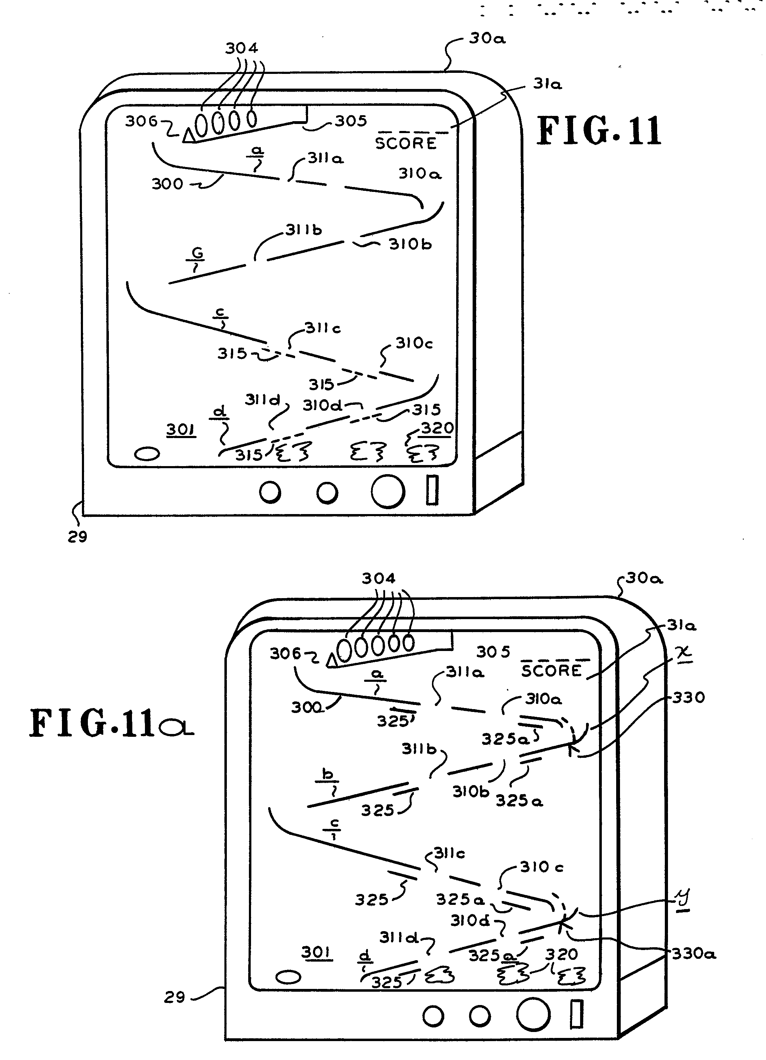

Fig. 11 and 11a are presented to show examples of layouts or graphics for electronic games that may be programmed in program storage devices, or ROM´s and played using electric signals corresponding to physiological or muscular response or movement for controlling the controllable images in the screen.

Fig. 11 shows a monitor or TV receiver 30a with a screen 31a on which is represented a track 300 which is segmented into four sections, a, b, c and d each section having opening which, when open, form a pitfall. The track 300 is pitched downward with respect to the top to bottom of the screen in a manner so as to zig zag across and down the screen. Each section of the track is separated so that an object/image, such as a ball, or an egg, for example, may travel or roll down the track making its way from one section to the next finally ending in a "home" or safe position 301.

The object of the game is to block off or close the openings of the pitfall as the moving image traverses the track so as to prevent the moving image from falling through the pitfall.

In the preferred graphs the moving image is an egg 304, four of which are illustrated stored in the storage rack 305 held by chock 306. The pitfall holes 310a and 311a and 310b and 311b of tracks sections a and b respectfully are illustrated open. The pitfall holes 310c and 310d and 311c and 311d are illustrated as blocked by the broken line bars 315. The bars 315 are controlled by the player, being displaced by the player-controlled signal from a blocking position whereby the lower pitfall holes 310b, 310c and 311d are blocked and the holes 310a and b and 311a and b are open, to a position where the blocking bars 315 block or close the upper pitfall holes 310a and 310b and 311a and 311b while the pitfall holes 310c and 310d and 311c and 311d are left open. The electric control signal for shifting the blocking bars may be a physiological response signal fed into the pre-processor module, processed and fed to the host computer for use on playing the game.

The release of the moving image, an egg, for example is under control of the host computer, as programmed in the ROM. The timing interval between the released eggs may be function of a second physiological response signal of the person playing the game. Should the moving egg 304, moving down the track 300 fall into any of the pitfall holes 310 or 311 which may not be covered by a bar 315, the egg will fall and smash, as at 320. An egg safe at home 301 may be used to score points.

Fig. 11a illustrates graphics for an alternate electronic game some what more complex than that disclosed in Fig. 11 but using a similar format. In this game the rolling or moving images, eggs, for example 304 are stored in the rack 305 and held by the chock 306. The track 300 is sectioned into sections a, b, c and d and are pitched downward and across the screen 31a on the monitor or TV receiver 30a. Each of the sections of the track include holes 310 and 311 forming pitfalls in the track. Associated with each hole are blocking bars 325 for holes 311a, 311b, 311c and 311d, bars 325a for holes 310a, 310b, 310c, and 310d. The normal position of the bars 325 and 325a is removed from the blocking position so as to expose the pitfall holes. In addition to pitfalls for the moving images, this electronic game provides cliff-falls by hinging, at 330 and 330a, the end portions of track sections b and d of the track 300. The hinged sections x and y close over the upper adjacent track end, forming cliff-fall when the bars 325 or 325a are moved into blocking position. This prevents keeping the blocking bars in blocking position while the moving image moves down the track.

Electronic games, such as those represented by the illustration of graphics, in Figs. 11 and 11a may be played by a person in conjunction with exercising while practicing the present invention. Some factors, such as the release of the moving images would be controlled by the computer while the timing may be a function of a selected characteristic of a selected physiological response, of the person so exercising, represented in the form of an electric signal. Displacement of the blocking bars as presented in Fig. 11 from one set of pitfall holes to the other set of pitfall holes may be electric signals representing a physiological response of a repeated, voluntary muscular action on the part of the person exercising.

The electronic game represented by the illustrated graphics in Fig. 11a may be played in similar manner except that the set of blocking bars 325 may be positioned in the pitfall holes 311 by an electric signal representing a voluntary movement or operation of one or more sets of muscles, for example, the left arm muscles and the companion blocking bars 325a may be positioned in the pitfall holes 310 by an electric signal representing voluntary movement or operation of one or more of another set of muscles, for example, the right arm muscles. Thus, it will be seen that exercise for any practical purpose may be used in coordination with playing an electronic game when practicing the present invention. It will be appreciated that other formats and/or graphics for electronic games and other displays may be used in association with the practice of the present invention.

This invention has been shown and described with reference to a represention of a preferred embodiment of the system and apparatus along with flow diagrams of a preferred program. Alternate embodiments of the system and apparatus have been shown and described and other alternate apparatus has been suggested, without limitation. A novel exercise apparatus which may serve as an exercise component usable in the system has been shown and described along with examples of electronic game graphs that may be used.

Although a two channel system has been described, expansion of the system into a system of three or more channels may be accomplished by those skilled in the art adhering to the principles taught herein. Other changes and substitutions may be made by those skilled in the art using the concept and principles of the invention disclosed herein without departing from the invention.

The various and sundry steps of a program to be written into a CPU 46 (Fig. 3) are set forth as follows:

INTERRUPT SERVICE ROUTINE FOR MICROCONDITIONER 8049

THIS ROUTINE EITHER ACCEPTS A COMMAND FROM THE HOST OR PROVIDES THE HOST WITH DATA DEPENDING ON THE STATE OF THE CMD(TO) PIN OF THE 8049. THE STATE OF THE CHNL(P22) PIN DETERMINES WHICH CHANNEL IS INVOLVED IN THE TRANSFER.

HANDSHAKING

FOR DATA TRANSFER FROM 8049 TO HOST - HOST RAISES INT PIN OF 8049. 8049 PLACES DATA ON BUS PORT THEN LOWERS ACK(P23) WHEN DATA IS VALID. HOST LOWERS INT PIN WHEN IT HAS ACCEPTED DATA. 8049 RAISES ACK TO ACKNOWLEDGE.

FOR COMMAND TRANSFER FROM HOST TO 8049 - HOST RAISES INT PIN OF 8049 AND PLACES COMMAND ON BUS PORT. 8049 READS BUS PORT THEN LOWERS ACK. HOST RELEASES INT PIN. 8049 RAISES ACK.

REGISTER USAGE

BANK 0 REGISTERS TO BE USED BY ROUTINE FOR CHANNEL 0
BANK 1 REGISTERS TO BE USED BY ROUTINE FOR CHANNEL 1
REGISTERS ARE ALLOCATED AS FOLLOWS:
R7 : USED BY INT ROUTINE TO STORE RO(BANK 0) AND ACC(BANK 1)
R6 : RESULT REGISTER OF PROCESS ROUTINE
R5 : USED BY PROCESS ROUTINES
R4 : SCALE PARAMETER (BITS 0, 1), APT PARAM (4, 5), CHANGE
     FLAG (BI TS 6, 7)
R3 : TIME PARAMETER
R2 : THRESHOLD PARAMETER
R1 : USED BY PROCESS ROUTINES
R0 : USED BY PROCESS ROUTINES

CHANGE FLAG = 00 = > INT TO READ DATA
CHANGE FLAG = 11 = > ROUTINE CODE OR PARAMETER CHANGED
CHANGE FLAG = 01 = > NO EXTERNAL INTERRUPT

ROUTINE CODE IS STORED IN MEM LOC 33 (CHANNEL 0) AND 34 (CHAN 1)

## COMMAND ROUTINES

THE FOLLOWING IS A LIST OF THE DIFFERENT ROUTINE CODES THAT
MAY BE TRANSFERRED FROM THE HOST. THIS ROUTINE WILL ACCEPT
THE CODES AND PROCESS THEM.

### ROUTINE CODES

| NAME | P21 | P20 | P17 | P16 | P15 | P14 | P13 | P12 | P11 | P10 | CODE |
|------|-----|-----|-----|-----|-----|-----|-----|-----|-----|-----|------|
| RESET | 0 | 0 | 0 | 0 | 0 | 0 | 0 | 0 | 0 | 0 | 0 |
| HALT | 0 | 0 | 0 | 0 | 0 | 0 | 0 | 0 | 0 | 1 | 1 |
| A/D | 0 | 0 | 0 | 0 | 0 | 0 | 0 | 0 | 1 | 0 | 2 |
| PEAK + | 0 | 0 | 0 | 0 | 0 | 0 | 0 | 0 | 1 | 1 | 3 |
| PEAK - | 0 | 0 | 0 | 0 | 0 | 0 | 0 | 1 | 0 | 0 | 4 |
| DIFF | 0 | 0 | 0 | 0 | 0 | 0 | 0 | 1 | 0 | 1 | 5 |
| EMG | 0 | 0 | 0 | 0 | 0 | 0 | 0 | 1 | 1 | 0 | 6 |
| EVT/T | 0 | 0 | 0 | 0 | 0 | 0 | 0 | 1 | 1 | 1 | 7 |
| T/EVT | 0 | 0 | 0 | 0 | 0 | 0 | 1 | 0 | 0 | 0 | 8 |
| TRIG | 0 | 0 | 0 | 0 | 0 | 0 | 1 | 0 | 0 | 1 | 9 |
| AVGAD | 0 | 0 | 0 | 0 | 0 | 0 | 1 | 0 | 1 | 0 | 10 |
| SET APT | 0 | 0 | 0 | 0 | 0 | 1 | 0 | 0 | X | X | 11 |
| SET SCL | 0 | 0 | 0 | 0 | 1 | 0 | X | X | X | X | 12 |
| SET TIME | 1 | 0 | X | X | X | X | X | X | X | X | 13 |
| SET THRS | 0 | 1 | X | X | X | X | X | X | X | X | 14 |
| SET I/O | 0 | 0 | 0 | 0 | 0 | 0 | 1 | 1 | X | X | 15-18 |

```
0700                   ORG    0700H        ;START OF INTERRUPT ROUTINE
0700 8603    ATN       JNI    YINT
0702 83                RET
0703 B87F    YINT      MOV    R0, No07FH   ;SET EOC FLAG
0705 B0FF              MOV    CdR0, NoOFFH
0707 0A                IN     A, P2        ;TEST WHICH CHANNEL
0708 5228              JB2    CN1
070A
070A C5                SEL    RB0
070B B870              MOV    R0, No70H    ;CH0 BUFFER POINTER
```

```
070D 10                 INC    CdR0      ;INC POINTER MOD AVGPTS TO
                                          LAST DATA
070E FC                 MOV    A, R4
070F 9217               JB4    B40       ;SET SIZE OF FFER
0711 B21D               JB5    B50
0713 2301               MOV    A, No01H
0715 E423               JMP    GSIZE0
0717 B221    B40        JB5    B5B40
0719 2303               MOV    A, No03H
071B E423               JMP    GSIZE0
071D 2307    B50        MOV    A, No07H
071F E423               JMP    GSIZE0
0721 230F    B5B40      MOV    A, NoOFH
0723 50      GSIZE0     ANL    A, CdR0
0724 4350               ORL    A, No050H
0726 E444               JMP    TCMD
0728 D5      CN1        SEL    RB1
0729 B874               MOV    R0, No74H  ;POINT TO CH1 AVG BUFFER
072B 10                 INC    CdR0      ;INC POINTER MOD AVGPTS TO
                                          LAST
072C FC                 MOV    A, R4
072D 9235               JB4    B41       ;SET SIZE OF BUFFER
072F B23B               JB5    B51
0731 2301               MOV    A, No01H
0733 E441               JMP    GSIZE1
0735 B23F    B41        JB5    B5B41
0737 2303               MOV    A, No03H
0739 E441               JMP    GSIZE1
073B 2307    B51        MOV    A, No07H
073D E441               JMP    GSIZE1
073F 230F    B5B41      MOV    A, NoOFH
0741 50      GSIZE1     ANL    A, CdR0
0742 4360               ORL    A, No060H
0744 265A    TCMD       JNT0   CMD       ;TEST IF COMMAND
```

OUTPUT DATA TO HOST

```
0746 D480              CALL   AVERG
0748 564E              JT1    NOEOC      ;IF EOC AT THIS POINT DONT
                                          WAIT LATER
074A B87F              MOV    RO, NoO7FH
074C B000              MOV    CdRO, NoOH ;CLEAR EOC FLAG
074E 9AF7    NOEOC     ANL    P2, NoOF7H ;LOWER ACK
0750 8650    LOOP1     JNI    LOOP1      ;WAIT FOR INT TO GO AWAY
0752 80                MOVX   A, CdRO    ;TRISTATE BUS
0753 8A08              ORL    P2, NoO8H  ;RAISE ACK
0755 FC                MOV    A, R4      ;RETURN CODE = READ
0756 533F              ANL    A, NoO3FH
0758 AC                MOV    R4, A
0759 93                RETR              ;RESTORE INTERRUPTED BANK
```

INPUT COMMAND OR PARAMETER FROM HOST

```
075A 0A      CMD       IN     A, P2      ;TEST IF SETTIME, SETTHRESH
075B 5303              ANL    A, NoO3H
075D 96CD              JNZ    TPMO       ;IF SO DETERMINE WHICH ONE
075F 08                INS    A, BUS     ;TEST IF SETAPT, SETSCALE
0760 5330              ANL    A, NoO30H  ;BITS 4, 5 ARE 1 FOR THESE
0762 9687              JNZ    SETPS      ;IF SO DETERMINE WHICH ONE
```

NOT SETTING PARAM. LOAD ROUTINE CODE

```
0764 0A                IN     A, P2      ;TEST WHICH LOCATION
0765 526B              JB2    M34
0767 B821              MOV    RO, NoO21H ;CHANNEL 0 USES LOC 33
0769 E46D              JMP    PUTSAMP
076B B822    M34       MOV    RO, NoO22H ;CHANNEL 1 USES LOC 34
076D 08      PUTSAMP   INS    A, BUS     ;GET LS 8 BIT ROUTINE CODE

076E 9AF7              ANL    P2, NoOF7H ;LOWER ACK
0770 5274              JB2    TRYB3
0772 E476              JMP    NCBITS
0774 72D9    TRYB3     JB3    CBITS
```

```
0776 A0    NCBITS   MOV    CdR0,A      ;LOAD ROUTINE CODE INTO RAM

0777 96EB           JNZ    DONE
0779 15    SRESET   DIS    I           ;DISABLE INTERRUPTS
077A 35             DIS    TCNTI
077B C7             MOV    A, PSW
077C 2301           MOV    A, No01H
077E D7             MOV    PSW, A
077F B808           MOV    R0, No08H   ;POINT TO TOP OF STACK
0781 B000           MOV    CdR0, NoOH  ;HAVE IT POINT TO 0
0783 18             INC    R0
0784 B000           MOV    CdR0, NoOH
0786 93             RETR
```

PARAMETER SET COMMANDS

```
0787 9AF7  SETPS    ANL    P2, NoOF7H  ;LOWER ACK
0789 9295           JB4    SETAPT      ;TEST IF SETAPT OR SETSCALE

078B 08    SETSC    INS    A, BUS
078C 530F           ANL    A, NoOFH
078E 2C             XCH    A, R4
078F 53F0           ANL    A, NoOFOH
0791 4C             ORL    A, R4
0792 AC             MOV    R4, A       ;SCALE PARAMETER
0793 E4EB           JMP    DONE
0795 08    SETAPT   INS    A, BUS
0796 5303           ANL    A, No03H    ;REMOVE ALL BUT PARAM FROM
                                        CODE
0798 47             SWAP   A
0799 2C             XCH    A, R4
079A 530F           ANL    A, NoOFH
079C 4C             ORL    A, R4
079D AC             MOV    R4, A       ;APT PARAMETER
079E C7             MOV    A, PSW      ;TEST WHICH CHANNEL
079F 92B7           JB4    AVCH1
07A1 B850           MOV    R0, No50H
07A3 B000  SAPL1    MOV    CdR0, No0
07A5 18             INC    R0
```

```
07A6 F8              MOV    A, R0
07A7 530F            ANL    A, No0FH
07A9 96A3            JNZ    SAPL1
07AB B870            MOV    R0, No70H
07AD B050            MOV    CdR0, No50H
07AF 18              INC    R0
07B0 B000            MOV    CdR0, No0H
07B2 18              INC    R0
07B3 B000            MOV    CdR0, No0H
07B5 E4EB            JMP    DONE
07B7 B860   AVCH1    MOV    R0, No60H
07B9 B000   SAPL2    MOV    CdR0, No0
07BB 18              INC    R0
07BC F8              MOV    A, R0
07BD 530F            ANL    A, No0FH
07BF 96B9            JNZ    SAPL2
07C1 B874            MOV    R0, No74H
07C3 B060            MOV    CdR0, No60H
07C5 18              INC    R0
07C6 B000            MOV    CdR0, No0H
07C8 18              INC    R0
07C9 B000            MOV    CdR0, No0H
07CB E4EB            JMP    DONE
07CD 9AF7   TPM0     ANL    P2, No0F7H   ;LOWER ASK
07CF 12D5            JB0    SETHR        ;TEST IF SETTIME OR
                                           SETTHRESH
07D1 08              INS    A, BUS
07D2 AB              MOV    R3, A        ;TIME PARAMETER
07D3 E4EB            JMP    DONE
07D5 08     SETHR    INS    A, BUS
07D6 AA              MOV    R2, A        ;THRESHOLD PARAMETER
07D7 E4EB            JMP    DONE


07D9 67     CBITS    RRC    A            ;PUT IO BIT PATTERNS IN
                                           D6, D7
07DA F6E0            JC     SETD6
07DC 9ABF            ANL    P2, No0BFH
07DE E4E2            JMP    TD7
07E0 8A40   SETD6    ORL    P2, No040H
```

```
07E2 67      TD7     RRC   A
07E3 F6E9            JC    SETD7
07E5 9A7F            ANL   P2, No07FH
07E7 E4EB            JMP   DONE
07E9 8A80    SETD7   ORL   P2, No080H

07EB 86EB    DONE    JNI   DONE          ;WAIT FOR INTERRUPT TO END
07ED 80              MOVX  A, CdR0        ;TRISTATE BUS PORT
07EE 8A08            ORL   P2, No08H      ;RAISE ACK
07F0 FC              MOV   A, R4          ;RETURN CODE = WRITE
07F1 43C0            ORL   A, No0C0H
07F3 AC              MOV   R4, A
07F4 93              RETR


0680                 ORG   0680H
0680 A0      AVERG   MOV   CdR0, A        ;STORE NEW POINTER VALUE
0681 A9              MOV   R1, A          ;GET DATA AT THIS LOCATION
0682 F1              MOV   A, CDR1
0683 37              CPL   A              ;SUBTRACT LAST FROM SUM
0684 18              INC   R0             ;POINT TO LSB
0685 97              CLR   C              ;SET CARRY
0686 A7              CPL   C
0687 70              ADDC  A, CdR0
0688 A0              MOV   CdR0, A
0689 F690            JC    NBOR           ;TEST IF BORROW
068B 18              INC   R0             ;POINT TO MSB
068C F0              MOV   A, CdR0
068D 07              DEC   A
068E A0              MOV   CdR0, A
068F C8              DEC   R0             ;POINT TO LSB
0690 FE      NBOR    MOV   A, R6          ;PUT NEW DATA IN BUFFER
0691 A1              MOV   CdR1, A

0692 60              ADD   A, CdR0        ;ADD NEW DATA TO SUM
0693 A0              MOV   CdR0, A
0694 E699            JNC   NCAR           ;TEST IF CARRY
0696 18              INC   R0             ;POINT TO MSB
0697 10              INC   CdR0
0698 C8              DEC   R0             ;POINT TO LSB
```

```
0699 FC      NCAR    MOV    A, R4
069A 729F            JB3    DOAVG        ;TEST IF AVERAGING DATA
069C FE              MOV    A, R6        ;NOT AVERAGING, JUST DUMP
                                          R6
069D 02              OUTL   BUS, A
069E 83              RET
069F B2AB    DOAVG   JB5    B5           ;TEST WHICH PART OF SUM TO
                                          TAKE
06A1 92C6            JB4    B4N5
06A3 18              INC    R0           ;POINT TO MSB
06A4 F0              MOV    A, CdR0
06A5 67              RRC    A            ;GET LS BIT IN CARRY
06A6 C8              DEC    R0
06A7 F0              MOV    A, CdR0      ;GET LSB
06A8 67              RRC    A            ;SEND BITS 1-8 OF SUM
06A9 02              OUTL   BUS, A
06AA 83              RET
06AB 92BE    B5      JB4    B4B5
06AD F0              MOV    A, CdR0      ;GET LSB
06AE 67              RRC    A            ;ROTATE 3 TIMES
06AF 67              RRC    A
06B0 67              RRC    A
06B1 531F            ANL    A, No01FH

06B3 A9              MOV    R1, A
06B4 13              INC    R0
06B5 F0              MOV    A, CdR0
06B6 77              RR     A
06B7 77              RR     A
06B8 77              RR     A
06B9 53E0            ANL    A, No0E0H
06BB 49              ORL    A, R1
06BC 02              OUTL   BUS, A
06BD 83              RET
06BE F0      B4B5    MOV    A, CdR0      ;BITS 4-7 OF LSB ARE LSN
06BF 18              INC    R0           ;POINT TO MSB
06C0 30              XCHD   A, CdR0      ;BITS 0-3 ARE MSN
06C1 47              SWAP   A            ;DATA IS CORRECT NOW
```

```
06C2 02              OUTL   BUS, A      ;DUMP DATA
06C3 47              SWAP   A           ;PUT MSN BACK
06C4 30              XCHD   A, CdRO
06C5 83              RET
06C6 F0       B4N5   MOV    A, CdRO     ;GET LSB
06C7 67              RRC    A
06C8 67              RRC    A
06C9 533F            ANL    A, NoO3FH
06CB A9              MOV    R1, A
06CC 18              INC    RO
06CD F0              MOV    A, CdRO
06CE 77              RR     A
06CF 77              RR     A
06D0 53C0            ANL    A, NoOCOH
06D2 49              ORL    A, R1
06D3 02              OUTL   BUS, A
06D4 83              RET
```

## MICROCONDITIONER SCHEDULER ROUTINE

THIS ROUTINE PERFORMS A/D FOR EACH CHANNEL, SETS UP 500 MICROSEC TIMER, AND CALLS PROCESSING ROUTINE FOR EACH CHANNEL

```
0000                 ORG    OH
0000 0409     ZERO   JMP    INIT        ;START OF SCHEDULER
0003                 ORG    03H
0003 E400            JMP    ATN         ;EXIT INTERRUPT VECTOR
0007                 ORG    07H
0007 84EB            JMP    TIMERI      ;TIMER INTERRUPT VECTOR
```

## INITIALIZATION

```
0009 C5       INIT   SEL    RBO
000A B87F            MOV    RO, NoO7FH  ;SET ALL OF RAM TO O
000C B000     NZ     MOV    CdRO, NoO
000E E80C            DJNZ   RO, NZ
0010 B870            MOV    RO, No70H
0012 B050            MOV    CdRO, No50H
0014 B874            MOV    RO, No74H
```

```
0016 B060              MOV    CdR0, No60H
0018 B87F              MOV    R0, No07FH
001A B0FF              MOV    CdR0, No0FFH

001C 80                MOVX   A, CdR0       ;TRISTATE BUS PORT
001D 89FF              ORL    P1, NoOFFH    ;PORT 1 INPUT
001F 8AFF              ORL    P2, NoOFFH    ;PORT 2 MIXED
0021 C5                SEL    RB0           ;INITIALIZE BANK 0
                                              REGISTERS
0022 BC40              MOV    R4, No040H    ;SCALE, AVGPT DEFAULT
0024 BB01              MOV    R3, No01H     ;TIME DEFAULT
0026 BA80              MOV    R2, No080H    ;THRESHOLD DEFAULT
0028 D5                SEL    RB1           ;INITIALIZE BANK 1
                                              REGISTERS
0029 BC40              MOV    R4, No040H    ;SCALE, AVGPT DEFAULT
002B BB01              MOV    R3, No01H     ;TIME DEFAULT
002D BA80              MOV    R2, No080H    ;THRESHOLD DEFAULT
002F 2301              MOV    A, No01H      ;STARTUP BOTH CHANNELS WITH
0031 B821              MOV    R0, No021H    ;ROUTINE CODE = HALT
0033 A0                MOV    CdR0, A
0034 18                INC    R0
0035 A0                MOV    CdR0, A
0036 23F5              MOV    A, No         ;11 TIMER TRICKS = 480
                              (OFFH-0AH)      MICROSEC
0038 62                MOV    T, A
0039 55                STRT   T             ;1 TIMER INT PER 500
                                              MICROSEC
003A 25                EN     TCNTI
003B 8A10              ORL    P2, No010H    ;RAISE SC FOR IST TIME
```

CHANNEL 0 PROCESSING, CHANNEL 1 SETUP

```
003D                                       ;SC HAS BEEN RAISED
003D
003D C5     CHNL0      SEL    RB0           ;CHANNEL 0 USES BANK 0
003E B820              MOV    R0, No020H    ;STORE SAMPLE IN DATA MEM
                                              LOC 32
0040 A0                MOV    CdR0, A
0041 B821              MOV    R0, No021H    ;PUT ROUTINE CODE IN R1
```

```
0043 F0              MOV     A, CdR0      ;FOR PROCESS ROUTINE
0044 A9              MOV     R1, A
0045 B830            MOV     R0, No30H    ;POINT TO CHANNEL 0 DATA
                                          REG
0047 9AEF            ANL     P2, NoOEFH   ;LOWER SC
0049 9ADF            ANL     P2, NoODFH   ;ADDR FOR CHANNEL 1
                                          SAMPLING
004B 147F            CALL    PROCESS      ;PROCESS CHANNEL 0 SAMPLE
004D F400            CALL    ATN
004F 564F    EOC0    JT1     EOC0         ;WAIT FOR EOC
0051 1473            CALL    WAIT         ;WAIT FOR DATA LINES TO
                                          SETTLE
0053 09              IN      A, P1        ;GET CHANNEL 1 SAMPLE
```

CHANNEL 1 PROCESSING, CHANNEL 0 SETUP

```
0054 8A10    CHNL1   ORL     P2, No010H   ;RAISE SC
0056 D5              SEL     RB1          ;CHANNEL 1 USES BANK 1
0057 B820            MOV     R0, No020H   ;STORE SAMPLE IN DATA MEM
                                          LOC 32
0059 A0              MOV     CdR0, A
005A B822            MOV     R0, No022H   ;PUT ROUTINE CODE IN R1
005C F0              MOV     A, CdR0      ;FOR PROCESS ROUTINE
005D A9              MOV     R1, A
005E B840            MOV     R0, No040H   ;POINT TO CHANNEL 0 DATA
                                          REGS
0060 9AEF            ANL     P2, NoOEFH   ;LOWER SC
0062 8A20            ORL     P2, No020H   ;ADDR FOR CHANNEL 0
                                          SAMPLING
0064 147F            CALL    PROCESS      ;PROCESS CHANNEL 1
                                          SAMPLE
0066 F400            CALL    ATN
0068
0068 5668    EOC1    JT1     EOC1         ;WAIT FOR EOC
006A 1473            CALL    WAIT         ;WAIT FOR OPTOS TO
                                          SETTLE
006C 09              IN      A, P1        ;GET CHANNEL 0 SAMPLE
006D 8A10            ORL     P2, No010H   ;RAISE SC
006F 168D    TIMERF  JTF     CHNL0        ;WAIT FOR TIMER INT
```

```
0071 046F                 JMP     TIMERF


                          DELAY ROUTINE


0073 B87F     WAIT        MOV     RO, No07FH  ;TEST EOC FLAG
0075 F8                   MOV     A, RO
0076 C67C                 JZ      NOWAIT
0078 B807                 MOV     RO, No07H
007A E87A     HERE        DJNZ    RO, HERE
007C B0FF     NOWAIT      MOV     CdRO, NoOFFH  ;SET EOC FLAG
007E 83                   RET


        PROCESS SUBROUTINE JUMPS TO ROUTINE CORRESPONDING TO
        TO CODE IN ACCUMULATOR


007F
007F FC       PROCESS     MOV     A, R4         ;TEST INTERRUPT RETURN CODE

0080 F7                   RLC     A             ;R C = 1 => NEW ROUTINE
                                                          OR PARAM
0081 F692                 JC      FIRST
0083 F9                   MOV     A,R1          ;R C = 0  => NO INT OR
                                                          DATA READ
0084 0387                 ADD     A, NoJMPTBL1
0086 B3                   JMPP    CdA
0087 A1       JMPTBL1     DB      CODE0         ;RESET
0088 A3                   DB      CODE1         ;HALT
0089 A5                   DB      CODE2         ;ATOD
008A A7                   DB      CODE3         ;PEAKP
008B A9                   DB      CODE4         ;PEAKM
008C AB                   DB      CODE5         ;DIFF
008D AD                   DB      CODE6         ;EMG
008E AF                   DB      CODE7         ;EVT/T
008F B1                   DB      CODE8         ;T/EVT
0090 B3                   DB      CODE9         ;TRIG
0091 B5                   DB      CODE10        ;AVGAD
```

```
0092 F9       FIRST      MOV   A,R1         ;FIRST TIME IN ROUTINE,
                                             DO SETUP
     0093 0396            ADD   A, NoJMPTBL2
     0095 B3             JMPP   CdA
0096 B7       JMPTBL2    DB    ICODE0
     0097 B9             DB    ICODE1
     0098 BB             DB    ICODE2
     0099 BD             DB    ICODE3
     009A BF             DB    ICODE4
009B C1                  DB    ICODE5
     009C C3             DB    ICODE6
     009D C5             DB    ICODE7
     009E C7             DB    ICODE8
     009F C9             DB    ICODE9
00A0 CB                  DB    ICODE10

     00A1 04CD  CODE0     JMP   RESET
     00A3 04E4  CODE1     JMP   HALT
     00A5 04EE  CODE2     JMP   ATOD
     00A7 2409  CODE3     JMP   PEAKP
00A9 2425      CODE4     JMP   PEAKM
     00AB 2446  CODE5     JMP   DIFF
     00AD 4400  CODE6     JMP   EMG
     00AF 640A  CODE7     JMP   EVTT
     00B1 644B  CODE8     JMP   TEVT
00B3 64B1      CODE9     JMP   TRIG
     00B5 8421  CODE10    JMP   AVGAD
     00B7 04CD  ICODE0    JMP   RESET
     00B9 04D4  ICODE1    JMP   IHALT
     00BB 04EE  ICODE2    JMP   ATOD
00BD 2400      ICODE3    JMP   IPEAKP
     00BF 241C  ICODE4    JMP   IPEAKM
     00C1 2438  ICODE5    JMP   IDIFF
     00C3 2469  ICODE6    JMP   IEMG
     00C5 44FB  ICODE7    JMP   IEVTT
00C7 643F      ICODE8    JMP   ITEVT
     00C9 64A6  ICODE9    JMP   ITRIG
     00CB 8400  ICODE10   JMP   IAVGAD
```

DATA PROCESSING ROUTINES

RESET

```
OOCD 15     RESET   DIS    I              ;DISABLE INTERRUPTS
OOCE 35             DIS    TCNTI
OOCF C7             MOV    A,PSW          ;RESET STACK POINTER
OODO 27             CLR    A
OOD1 D7             MOV    PSW, A
OOD2 0400           JMP    ZERO           ;START ENTIRE PGM OVER
```

HALT

```
OOD4 FB     IHALT   MOV    A, R3          ;TIME PARAM=67 => SEND MSG
OOD5 D343           XRL    A, No67
OOD7 96E3           JNZ    IHD
OOD9 B930           MOV    R1, No30H
OODB B100           MOV    CdR1, NoO
OODD B940           MOV    R1, No40H
OODF B120           MOV    CdR1, No20H
OOE1 BDFF           MOV    R5, No255
OOE3 83     IHD     RET
OOE4 FB     HALT    MOV    A, R3
OOE5 D343           XRL    A, No67
OOE7 96EB           JNZ    HD2
OOE9 A43D           JMP    BODYLG
OOEB BE10   HD2     MOV    R6, NoO10H, ;RESULT <- VERSION No
OOED 83             RET
```

A TO D

```
OOEE B920   ATOD    MOV    R1, NoO20H     ;POINT TO SAMPLE
OOFO F1             MOV    A, CdR1
OOF1 9453           CALL   SSCALE         ;SCALE SAMPLE IN A
OOF3 AE             MOV    R6,A           ;LOAD RESULT W/ SAMPLE
COF4 B3             RET

0500                ORG    500H
```

```
0500 8080A88A        DB      128,128,168,138,138,128,138,138,128,
                             138,138,168
050C 808A768A        DB      128,138,118,138,128,168,128,138,138,
                             128,138,138
0518 8A4E4E4E        DB      138,78,78,78,64
0520                 ORG     520H
0520 7F7F6B6B        DB      127,127,107,107,107,127,107,107,127,
                             107,107,107
052C 7F6B6B4E        DB      127,107,107,78,127,107,127,107,107,
                             127,107,107
0538 4E4E4E4E        DB      78,78,78,78,64

053D FD    BODYLG    MOV     A,R5
053E 9644            JNZ     BODY2

0540 BDFF            MOV     R5, No255
0542 10              INC     CdR0
0543 83              RET
0544 CD    BODY2     DEC     R5
0545 F0              MOV     A, CdR0
0546 A3              MOVP    A, CdA
0547 D340            XRL     A, No64
0549 964D            JNZ     BODYD
054B 04D4            JMP     IHALT
054D F0    BODYD     MOV     A, CdR0
054E A3              MOVP    A, CdA
054F AE              MOV     R6, A
0550 83              RET

0580                 ORG     580H
0580 434F5059        DB      'COPYRITE (C) 1985'
0591 424F4459        DB      'BODYLOG INCORPORATED'


                             PEAK PLUS


0100                 ORG     100H
0100 BE00   IPEAKP   MOV     R6, No0H     ;RESET PEAK
0102 FC              MOV     A,R4         ;RESET WRITE BIT
0103 537F            ANL     A, No07FH    ;AND SET NONREAD BIT
```

```
0105 4340          ORL     A, No040H
0107 AC            MOV     R4, A
0108 83            RET
0109 FC     PEAKP  MOV     A, R4      ;TEST IF DATA WAS READ
010A F7            RLC     A
010B F7            RLC     A
010C E600          JNC     IPEAKP     ;RESET PEAK AFTER READ
010E B920          MOV     R1, No020H ;GET SAMPLE
0110 F1            MOV     A, CdR1
0111 9453          CALL    SSCALE
0113 37            CPL     A          ;TEST IF A < R6
0114 6E            ADD     A, R6      ;R6 CONTAINS CURRENT PEAK
0115 F61B          JC      PPDONE
0117 F1            MOV     A, CdR1    ;SAMPLE IS BIGGER, PUT IN
R6
0118 9453          CALL    SSCALE
001A AE            MOV     R6, A
011B 83     PPDONE RET


                        PEAK MINUS


011C
011C BEFF   IPEAKM MOV     R6, NoOFFH ;RESET TROUGH
011E FC            MOV     A, R4
011F 537F          ANL     A, No07FH  ;RESET WRITE BIT
0121 4340          ORL     A, No040H  ;AND SET NONREAD BIT
0123 AC            MOV     R4, A
0124 83            RET
0125 FC     PEAKM  MOV     A, R4      ;TEST IF DATA WAS READ
0126 F7            RLC     A
0127 F7            RLC     A
0128 E61C          JNC     IPEAKM     ;RESET TROUGH AFTER READ
012A B920          MOV     R1, No20H  ;GET SAMPLE
012C F1            MOV     A, CdR1
012D 9453          CALL    SSCALE
012F 37            CPL     A          ;TEST IF A > = R6
0130 6E            ADD     A, R6
0131 E637          JNC     PMDONE
```

```
      0133 F1                    MOV    A, CdR1      ;SAMPLE IS SMALLER PUT IN
                                                             R6
      0134 9453                  CALL   SSCALE
      0136 AE                    MOV    R6, A
      0137 83      PMDONE        RET


                              PEAK DIFFERENCE


      0138 BE00    IDIFF        MOV    R6, No00H    ;RESET DIFF
      013A B000                 MOV    CdR0, No00H  ;RESET PEAK
      013C 18                   INC    R0
      013D B0FF                 MOV    CdR0, NoOFFH ;RESET TROUGH
      013F FC                   MOV    A, R4        ;RESET WRITE BIT
      0140 537F                 ANL    A, No07FH    ;AND SET NONREAD BIT
      0142 4340                 ORL    A, No040H
      0144 AC                   MOV    R4, A
      0145 83                   RET
      0146 FC      DIFF         MOV    A, R4        ;TEST IF DATA WAS READ
      0147 F7                   RLC    A
      0148 F7                   RLC    A
      0149 E638                 JNC    IDIFF
      014B B920                 MOV    R1, No20H    ;GET SAMPLE
      014D F1                   MOV    A, CdR1
      014E 37                   CPL    A            ;TEST IF A < PEAK
      014F 60                   ADD    A, CdR0      ;ADD TO PEAK
      0150 F654                 JC     TRYTROU
      0152 F1                   MOV    A, CdR1      ;SAMPLE IS BIGGER, PUT IN
                                                             PEAK
      0153 A0                   MOV    CdR0, A
      0154 18      TRYTROU      INC    R0           ;POINT TO TROUGH
      0155 F1                   MOV    A, CdR1
      0156 37                   CPL    A            ;TEST IF A > = TROUGH
      0157 60                   ADD    A, CdR0
      0158 E65C                 JNC    DDONE
      015A F1                   MOV    A, CdR1      ;SAMPLE IS SMALLER, PUT IN
                                                             TROUGH
      015B A0                   MOV    CdR0, A
      015C C8      DDONE        DEC    R0           ;POINT TO PEAK
      015D F0                   MOV    A, CdR0      ;PUT PEAK IN A
```

```
015E 37                CPL     A               ;SUBTRACT TROUGH FROM A
015F 18                INC     R0              ;POINT TO TROUGH
0160 60                ADD     A, CdR0
0161 37                CPL     A
0162 B920              MOV     R1, No020H      ;PUT UNSCALED No IN SAMPLE
                                                                    AREA
0164 A1                MOV     CdR1, A         ;WHERE IT CAN BE SCALED
0165 9453              CALL    SSCALE
0167 AE                MOV     R6, A           ;PUT DIFF IN RESULT
                                                          REGISTER
0168 83                RET


                          EMG


0169 FC        IEMG    MOV     A, R4           ;RESET WRITE BIT
016A 537F              ANL     A, No07FH
016C AC                MOV     R4, A
016D FB                MOV     A, R3           ;TEST TIME PARAMETER
016E 9677              JNZ     TRY100

0170 B064              MOV     CdR0, No100     ;TIME=0 => 50 SEC
0172 18                INC     R0              ; = 100  *  500 USEC
0173 B000              MOV     CdR0, No0
0175 2493              JMP     ZSUM

0177 07        TRY100  DEC     A
0178 9681              JNZ     TRY150          ;TEST TIME PARAMETER

017A B0C8              MOV     CdR0, No200     ;TIME=1 => 100 MSEC
017C 18                INC     R0              ; = 200 * 500 USEC
017D B000              MOV     CdR0,   No0
017F 2493              JMP     ZSUM

0181 07        TRY150  DEC     A               ;TEST TIME PARAMETER
0182 968B              JNZ     TRY200

0184 B00A              MOV     CdR0, No10      ;TIME=2 => 133 MSEC
0186 18                INC     R0              ;  = 266 * 500 USEC
0187 B000              MOV     CdR0, No0
```

```
    0189 2493              JMP     ZSUM

    018B 07      TRY200    DEC     A            ;TEST IF TIME PARAM
    018C 9699              JNZ     EIEMG        ;IS TOO BIG
    018E
    018E B090              MOV     CdR0, No144  ;TIME= 3 => 200 MSEC
    0190 18                INC     R0           ; = 400 * 500 USEC
    0191 B001              MOV     CdR0, No1

    0193 18      ZSUM      INC     R0           ;ZERO LOW & HI ORDER SUM
    0194 B000              MOV     CdR0, No0
    0196 18                INC     R0
    0197 B000              MOV     CdR0, No0
    0199 83      EIEMG     RET

    0200                   ORG     200H
    0200 F0      EMG       MOV     A, CdR0      ;TEST IF LAST SAMPLE WAS
                                                        TAKEN
    0201 18                INC     R0           ;POINT TO MS BYTE OF
                                                   SAMPLES
    0202 9609              JNZ     RECTIFY
    0204 F0                MOV     A, CdR0
    0205 9609              JNZ     RECTIFY
    0207 4426              JMP     EDUMP2
    0209 C8      RECTIFY   DEC     R0           ;FIRST D.P. DEC OF No
                                                   SAMPLES
    020A F0                MOV     A, CdR0
    020B 03FF              ADD     A, NoOFFH
    020D A0                MOV     CdR0, A
    020E 18                INC     R0
    020F F614              JC      ENOINC1
    0211 F0                MOV     A, CdR0
    0212 07                DEC     A
    0213 A0                MOV     CdR0, A
    0214 B920    ENOINC1   MOV     R1, No020H   ;POINT TO SAMPLE
    0216 F1                MOV     A, CdR1
    0217 F7                RLC     A            ;TEST IF OVER 127
    0218 F1                MOV     A, CdR1      ;RELOAD A; CY NOT CHANGED
    0219 F61C              JC      POS
```

```
021B 37              CPL    A
021C 537F    POS     ANL    A, No07FH
021E 18              INC    R0        ;POINT TO LS BYTE OF SUM
021F 60              ADD    A, CdR0   ;DP ADD OF RECTIFIED SAMP
                                       TO SUM

0220 A0              MOV    CdR0, A
0221 18              INC    R0        ;POINT TO MS BYTE OF SUM
0222 E625            JNC    ENOINC2   ;ADD CY INTO MS BYTE IF
                                       NEEDED

0224 10              INC    CdR0
0225 83      ENOINC2 RET
0226 FB      EDUMP2  MOV    A, R3     ;PUT SCALE FACTOR FOR TIME
                                       PARAM IN R6

0227 3233            JB1    GE2       ;SCALE FACTOR IS SAME FOR
                                       PARAM = 2,3

0229 122F            JB0    EQ1
022B BE02            MOV    R6, No2   ;PARAM= 0 => SCALE
                                       SUBFACTOR = 2

022D 4435            JMP    ADSC
022F BE01    EQ1     MOV    R6, No1   ;PARAM= 1 => SCALE
                                       SUBFACTOR = 1

0231 4435            JMP    ADSC
0233 BE00    GE2     MOV    R6, No0   ;PARAM = 2 => SCALE
                                       SUBFACTOR = 0


0235 FC      ADSC    MOV    A, R4     ;ADD SCALE PARAM TO SCALE
                                       FACTOR

0236 5307            ANL    A, No07H
0238 6E              ADD    A, R6
0239 AE              MOV    R6, A     ;PUT 16 BIT SCALE FACTOR
                                       IN R6


023A 18              INC    R0        ;POINT TO LSB OF SUM
023B F0              MOV    A, CdR0
023C AD              MOV    R5, A     ;PUT LSB IN R5
023D 18              INC    R0        ;POINT TO MSB OF SUM
023E F0              MOV    A, CdR0
023F A8              MOV    R0, A     ;PUT MSB IN R0
```

```
       0240 FB               MOV    A, R3        ;IF SCALE FACTOR = 2 THAN
                                                   MUST MULT BY 1.5

       0241 07               DEC    A
       0242 07               DEC    A
       0243 9656             JNZ    TSF
       0245 97               CLR    C            ;ROTATE RIGHT MSB
       0246 F8               MOV    A, R0
       0247 67               RRC    A
       0248 A9               MOV    R1, A        ;SAVE HALVED MSB
       0249 FD               MOV    A, R5
       024A 67               RRC    A            ;ROTATE CY INTO LSB
       024B 6D               ADD    A, R5        ;ADD HALVED LSB AND LSB
       024C AD               MOV    R5, A        ;SAVE IT AS LSB
       024D F9               MOV    A, R1        ;NOW DO MSB
       024E 78               ADDC   A, R0        ;INCLUDE CARRY FROM LSB ADD
       024F A8               MOV    R0, A
       0250 E656             JNC    TSF
       0252 B8FF             MOV    R0, NoOFFH
       0254 BDFF             MOV    R5, NoOFFH


       0256 FE      TSF      MOV    A, R6        ;ACT UPON SCALE FACTOR
       0257 965F             JNZ    TSF2
       0259 54E0             CALL   DPROTL       ;SCALE FACTOR = 0
       025B F6F7             JC     OVERFLO
       025D 44C7             JMP    EMGDONE


       025F 07      TSF2     DEC    A
       0260 9668             JNZ    TSF3
       0262 54E8             CALL   DPROTL2      ;SCALE FACTOR = 1
       0264 F6F7             JC     OVERFLO

       0266 44C7             JMP    EMGDONE


       0268 07      TSF3     DEC    A
       0269 9675             JNZ    TSF4
       026B 54E8             CALL   DPROTL2      ;SCALE FACTOR = 2
       026D F6F7             JC     OVERFLO
       026F 54E0             CALL   DPROTL
       0271 F6F7             JC     OVERFLO
```

```
0273 44C7              JMP      EMGDONE


0275 07      TSF4      DEC      A
0276 967E              JNZ      TSF5
0278 54CD              CALL     MIDNIB      ;SCALE FACTOR = 3
027A F6F7              JC       OVERFLO
027C 44C7              JMP      EMGDONE


027E 07      TSF5      DEC      A
027F 968B              JNZ      TSF6
0281 54E0              CALL     DPROTL      ;SCALE FACTOR = 4
0283 F6F7              JC       OVERFLO
0285 54CD              CALL     MIDNIB
0287 F6F7              JC       OVERFLO
0289 44C7              JMP      EMGDONE


028B 07      TSF6      DEC      A
028C 9698              JNZ      TSF7
028E 54E8              CALL     DPROTL2     ;SCALE FACTOR = 5
0290 F6F7              JC       OVERFLO
0292 54CD              CALL     MIDNIB
0294 F6F7              JC       OVERFLO
0296 44C7              JMP      EMGDONE


0298 07      TSF7      DEC      A
0299 96A9              JNZ      TSF8
029B 54E8              CALL     DPROTL2     ;SCALE FACTOR = 6
029D F6F7              JC       OVERFLO
029F 54E0              CALL     DPROTL
02A1 F6F7              JC       OVERFLO
02A3 54CD              CALL     MIDNIB
02A5 F6F7              JC       OVERFLO
02A7 44C7              JMP      EMGDONE


02A9 07      TSF8      DEC      A
02AA 96B2              JNZ      TSF9
02AC F8               MOV      A, R0       ;SCALE FACTOR = 7
02AD 96F7              JNZ      OVERFLO
02AF FD               MOV      A, R5
```

```
02B0 44C7              JMP    EMGDONE

02B2 07       TSF9     DEC    A
02B3 96BF              JNZ    TSF10
02B5 54E0              CALL   DPROTL        ;SCALE FACTOR = 8
02B7 F6F7              JC     OVERFLO
02B9 A8                MOV    R0, A
02BA 96F7              JNZ    OVERFLO
02BC FD                MOV    A, R5
02BD 44C7              JMP    EMGDONE

02BF 54E8     TSF10    CALL   DPROTL2
02C1 F6F7              JC     OVERFLO
02C3 A8                MOV    R0, A
02C4 96F7              JNZ    OVERFLO
02C6 FD                MOV    A, R5

02C7 AE       EMGDONE  MOV    R6, A
02C8 FC       OVDONE   MOV    A, R4         ;SET WRITE BIT SO THAT IEMG
                                            IS CALLED NEXT
02C9 4380              ORL    A, No080H
02CB AC                MOV    R4, A
02CC 83                RET

02CD F8       MIDNIB   MOV    A, R0         ;JOIN LO NIB OF HI BYTE
                                            & HI NIB OF LO BYTE
02CE 53F0              ANL    A, No0F0H
02D0 96DD              JNZ    OVFLO
02D2 F8                MOV    A, R0
02D3 47                SWAP   A
02D4 53F0              ANL    A, No0F0H
02D6 AE                MOV    R6, A
02D7 FD                MOV    A, R5
02D8 47                SWAP   A
02D9 530F              ANL    A, No0FH
02DB 4E                ORL    A, R6
02DC 83                RET
02DD 97       OVFLO    CLR    C
02DE A7                CPL    C
```

```
02DF 83              RET
02E0
02E0 FD    DPROTL    MOV     A, R5        ;DP ROTATE ROUTINE
                                          (R0) <- (R5) THRU A
02E1 97              CLR     C
02E2 F7              RLC     A
02E3 AD              MOV     R5, A
02E4 F8              MOV     A, R0
02E5 F7              RLC     A            ;TEST CARRY AFTER RET FOR
                                          OVERFLOW
02E6 A8              MOV     R0, A
02E7 83              RET


02E8 FD    DPROTL2   MOV     A, R5        ;DP 2BIT ROTATE
02E9 97              CRL     C
02EA F7              RLC     A
02EB 28              XCH     A, R0
02EC F7              RLC     A
02ED F6F6            JC      OVRFLO
02EF 28              XCH     A, R0


02F0 97              CLR     C
02F1 F7              RLC     A
02F2 AD              MOV     R5, A
02F3 F8              MOV     A, R0
02F4 F7              RLC     A
02F5 A8              MOV     R0, A
02F6 83    OVRFLO    RET


02F7 BEFF  OVERFLO   MOV     R6, NoOFFH
02F9 44C8            JMP     OVDONE



                 EVENT PER UNIT TIME


02FB B000  IEVTT     MOV     CdR0, NoOH   ;RESET COUNT LS BYTE
02FD 18              INC     R0
02FE B000            MOV     CdR0, NoOH   ;RESET COUNT MS BYTE
0300 18              INC     R0
0301 B000            MOV     CdR0, NoOH   ;RESET EVENT BEGIN FLAG
```

```
0303 BD00          MOV    R5, No0H      ;RESET No EVENTS
0305 FC            MOV    A, R4
0306 537F          ANL    A, No07FH     ;RESET WRITE BIT
0308 AC            MOV    R4, A
0309 83            RET
030A 18     EVTT   INC    R0            ;POINT TO MS COUNT
030B F0            MOV    A, CdR0       ;TEST IF COUNT = TIME PARAM
030C DB            XRL    A, R3
030D 9613          JNZ    ETNEQ

030F FD            MOV    A, R5         ;LOAD RESULT REG W/ No
                                         EVENTS
0310 AE            MOV    R6, A
0311 44FB          JMP    IEVTT         ;RESET EVERYTHING BUT
                                         RESULT

0313 C8     ETNEQ  DEC    R0            ;POINT TO COUNT LS BYTE
0314 10            INC    CdR0          ;INC LOW ORDER COUNTER
0315 F0            MOV    A, CdR0       ;TEST IF = 200
0316 D3C8          XRL    A, No200
0318 961F          JNZ    TTHRESH
031A 18            INC    R0
031B 10            INC    CdR0          ;INCREMENT MS COUNTER
031C C8            DEC    R0
031D B000          MOV    CdR0, No0H    ;RESET LOW ORDER COUNT

031F 18     TTHRESH INC   R0            ;POINT TO BEGIN EVENT FLAG
0320 18            INC    R0
0321 F0            MOV    A, CdR0
0322 9630          JNZ    NEED0
0324 B920          MOV    R1, No20H     ;GET SAMPLE
0326 F1            MOV    A, CdR1
0327 9453          CALL   SSCALE
0329 37            CPL    A             ;TEST IF > = THRESHOLD
032A 6A            ADD    A, R2
032B F62F          JC     NOTEVT

032D B0FF          MOV    CdR0, NoOFFH
032F 83     NOTEVT RET
```

```
0330 B920    NEEDO    MOV    R1, No20H    ;GET SAMPLE
0332 F1               MOV    A, CdR1
0333 9453            CALL    SSCALE
0335 74EC            CALL    HYSTER

0337 37              CPL     A            ;TEST IF > = THRESHOLD
0338 6A              ADD     A, R2
0339 E63E            JNC     NTDOWN
033B 1D              INC     R5           ;WENT DOWN, INC No EVENTS
033C B000            MOV     CdRO, NoOH   ;RESET BEGIN EVENT FLAG
033E 83     NTDOWN   RET
033F
```

## TIME PER UNIT EVENT

```
033F B000    ITEVT    MOV    CdRO, NoOH   ;RESET MS TIME COUNT
B341 18               INC    RO
0342 B000            MOV     CdRO, NoOH   ;RESET EVENT FLAG
0344 BD00            MOV     R5, NoOH     ;RESET LS TIME COUNT
0346 FC              MOV     A, R4
0347 537F            ANL     A, No07FH    ;RESET WRITE BIT
0349 AC              MOV     R4, A
034A 83              RET
034B 18     TEVT     INC     RO           ;POINT TO EVENT FLAG
034C F0              MOV     A, CdRO
034D 965B            JNZ     TTRY1        ;TEST IF WAITING FOR 1

034F B920            MOV     R1, No20H    ;GET SAMPLE
0351 F1              MOV     A, CdR1
0352 9453            CALL    SSCALE
0354 37              CPL     A            ;TEST IF STILL NO THRESHOLD
                                          (0)
0355 6A              ADD     A, R2
0356 F65A            JC      STILLO
0358 10              INC     CdRO         ;EVENT FLAG < - 1
0359 83              RET
035A 83     STILLO   RET
```

```
035B 07      TTRY1    DEC    A              ;TEST IF ADDING PULSES
035C 9685             JNZ    TTRY2

035E 1D              INC    R5             ;INC LS COUNT
035F FD              MOV    A, R5
0360 DB              XRL    A, R3
0361 9676            JNZ    NOT256         ;TEST FOR CARRY
0363 BD00            MOV    R5, NoOH       ;RESET LS COUNT
0365 C8              DEC    R0             ;POINT TO MS COUNT
0366 F0              MOV    A, CdR0
0367 D3FF            XRL    A, NoOFFH      ;DON'T INC TO WRAP AROUND
0369 18              INC    R0             ;POINT TO EVENT FLAG
036A C671            JZ     DPOVFLO
036C C8              DEC    R0             ;POINT TO MS COUNT
036D 10              INC    CdR0           ;NOT 255 YET, SO INC
036E 18              INC    R0             ;POINT TO EVENT FLAG
036F 6476            JMP    NOT256
0371 BEFF   DPOVFLO  MOV    R6, NoOFFH
0373 C8              DEC    R0             ;POINT TO MS COUNT
0374 643F            JMP    ITEVT
0376 00     NOT256   NOP

0377 B920            MOV    R1, No20H      ;GET SAMPLE
0379 F1              MOV    A, CdR1
037A 9453            CALL   SSCALE

037C 74EC            CALL   HYSTER
037E 37              CPL    A              ;TEST IF STILL THRESHOLD
037F 6A              ADD    A, R2
0380 E684            JNC    STIL1
0382 10              INC    CdR0           ;EVENT FLAG < - 3
0383 83              RET
0384 83     STIL1    RET

0385 1D     TTRY2    INC    R5             ;INC LS COUNT
0386 FD              MOV    A, R5
0387 DB              XRL    A, R3
0388 9696            JNZ    NT256          ;TEST FOR CARRY
038A BD00            MOV    R5, NoOH       ;RESET LS COUNT
```

```
038C C8              DEC    R0            ;POINT TO MS COUNT
038D F0              MOV    A, CdR0
038E D3FF            XRL    A, NoOFFH     ;DON'T INC TO WRAP AROUND
0390 18              INC    R0            ;POINT TO EVENT FLAG
0391 C671            JZ     DPOVFLO
0393 C8              DEC    R0            ;POINT TO MS COUNT
0394 10              INC    CdR0          ;NOT 255 YET CAN INC
0395 18              INC    R0            ;POINT TO EVENT FLAG
0396 00      NT256   NOP

0397 B920            MOV    R1, No20H     ;GET SAMPLE
0399 F1              MOV    A, CdR1
039A 9453            CALL   SSCALE
039C 37              CPL    A             ;TEST IF STILL NOT
                                            THRESHOLD
039D 6A              ADD    A, R2
039E F6A5            JC     STILO
03A0 C8              DEC    R0            ;POINT TO MS COUNT
03A1 F0              MOV    A, CdR0       ;MOVE IT TO RESULT REG
03A2 AE              MOV    R6, A
03A3 643F            JMP    ITEVT
03A5 83      STILO   RET


                     TRIGGER


03A6 BD00    ITRIG   MOV    R5, NoOH      ;RESET EVENT FLAG
03A8 BE00            MOV    R6, NoOH      ;RESET RESULT REG
03AA FC              MOV    A, R4
03AB 537F            ANL    A, No07FH     ;RESET WRITE BIT
03AD 4340            ORL    A, No040H     ;SET NON READ BIT
03AF AC              MOV    R4, A
03B0 83              RET


03B1 FD      TRIG    MOV    A, R5         ;TEST IF WAITING FOR NO
                                            THRESH
03B2 96C0            JNZ    TRTRY1

03B4 B920            MOV    R1, No20H     ;GET SAMPLE
03B6 F1              MOV    A, CdR1
```

```
03B7 9453              CALL    SSCALE
03B9 37                CPL     A              ;TEST IF STILL THRESH
03BA 6A                ADD     A, R2
03BB E6BF              JNC     STILLL1
03BD 1D                INC     R5
03BE 83                RET
03BF 83     STILLL1    RET


03C0 07     TRTRY1     DEC     A              ;TEST IF WAITING FOR
                                                 THRESH
03C1 96CF              JNZ     TRTRY2

03C3 B920              MOV     R1, No20H      ;GET SAMPLE
03C5 F1                MOV     A, CdR1
03C6 9453              CALL    SSCALE
03C8 37                CPL     A              ;TEST IF STILL NOTHRESH
03C9 6A                ADD     A, R2
03CA F6CE              JC      STILLL0
03CC 1D                INC     R5
03CD 83                RET
03CE 83     STILLL0    RET


03CF 07     TRTRY2     DEC     A              ;TEST IF WAITING FOR
                                                 NOTHRESH
03D0 96E6              JNZ     TRTRY3

03D2 B920              MOV     R1, No20H      ;GET SAMPLE
03D4 F1                MOV     A, CdR1
03D5 9453              CALL    SSCALE
03D7 74EC              CALL    HYSTER
03D9 37                CPL     A              ;TEST IF STILL THRESH
03DA 6A                ADD     A, R2
03DB E6E5              JNC     STL1
03DD 1D                INC     R5
03DE BEFF              MOV     R6, NoOFFH     ;LOAD RESULT REG W/ TRIG
03E0 FC                MOV     A, R4

03E1 4340              ORL     A, No040H      ;SET NON READ BIT
03E3 AC                MOV     R4, A
```

```
03E4 83              RET
03E5 83       STL1   RET


03E6 FC       TRTRY3 MOV    A, R4      ;WAIT TILL THIS TRIG IS
                                        READ
03E7 F7              RLC    A          ;TEST IF DATA WAS READ
03E8 F7              RLC    A
03E9 E6A6            JNC    ITRIG      ;RESET TRIG IF DATA WAS
                                        READ

03EB 83              RET
```

ROUTINE TO ADD 1/8 OF THRESHOLD TO SAMPLE TO GIVE HYSTERESIS

```
03EC A9       HYSTER MOV    R1, A      ;SAVE SCALED SAMPLE
03ED FA              MOV    A, R2      ;GET THRESHOLD
03EE 77              RR     A          ;DIVIDE BY 8
03EF 77              RR     A
03F0 77              RR     A
03F1 531F            ANL    A, No01FH
03F3 69              ADD    A, R1      ;ADD 1/8 THRESH & SAMPLE
03F4 E6F8            JNC    NOF        ;TEST FOR OVERFLOW
03F6 23FF            MOV    A, NoOFFH
03F8 83       NOF    RET
```

                         AVERAGING A TO D


                VARIABLE ASSIGNMENTS
                CdRO = LOW ORDER SUM
                1 + CdRO = HI ORDER SUM
                R5 =  NoPTS

```
0400                 ORG    0400H
0400 B000     IAVGAD MOV    CdRO, NoOH ;RESET SUM
0402 18              INC    RO
0403 B000            MOV    CdRO, NoOH
0405 FC              MOV    A, R4
0406 537F            ANL    A, No07FH  ;RESET WRITE BIT
0408 AC              MOV    R4, A
0409 FC       GNPTS  MOV    A, R4      ;GET AVGPTS PARAM
```

```
040A 47                  SWAP   A
040B 5303         ANL    A, No03H
040D 9611         JNZ    ATRY1      ;TEST IF PARAM = 0
040F BD11         MOV    R5, No11H
0411 07    ATRY1  DEC    A          ;TEST IF PARAM = 1
0412 9616         JNZ    ATRY2
0414 BD21         MOV    R5, No21H
0416 07    ATRY2  DEC    A          ;TEST IF PARAM = 2
0417 961B         JNZ    ATRY3
0419 BD41         MOV    R5, No41H
041B 07    ATRY3  DEC    A          ;TEST IF PARAM = 3
041C 9620         JNZ    IADONE
041E BD81         MOV    R5, No081H
0420 83    IADONE RET


0421 ED47  AVGAD  DJNZ   R5, ANLAST ;TEST IF LAST No WAS TAKEN

0423 BDFB         MOV    R5, NoOFBH ;COMPUTE SHIFT FACTOR
0425 FC           MOV    A, R4      ; = 4 - AVGPTS
0426 47           SWAP   A
0427 5303         ANL    A, No03H
0429 6D           ADD    A, R5
042A 37           CPL    A
042B AD           MOV    R5, A      ;PUT SCALE FACTOR IN R5
042C F0           MOV    A, CdR0    ;PUT SUM LS BYTE IN R1
042D A9           MOV    R1, A
042E B000         MOV    CdR0, NoOH ;RESET LS BYTE
0430 18           INC    R0
0431 F0           MOV    A, CdR0    ;PUT SUM MS BYTE IN A
0432 B000         MOV    CdR0, NoOH  ;RESET MS BYTE
0434 A8           MOV    R0, A       ;PUT SUM MS BYTE IN R0
0435 F9   ASHIFTS MOV    A, R1      ;D.P. ROTATE
0436 F7           RLC    A

0437 A9           MOV    R1, A
0438 F8           MOV    A, R0
0439 F7           RLC    A
043A A8           MOV    R0, A
043B F643         JC     AOVRFLO    ;TEST FOR OVERFLOW
```

```
043D ED35            DJNZ  R5, ASHIFTS
043F F8              MOV   A, RO        ;PUT SHIFTED RESULT IN
                                         RESULT REG

0440 AE              MOV   R6, A
0441 8409            JMP   GNPTS        ;COMPUTE NoPTS
0443 BEFF  AOVRFLO   MOV   R6, NoOFFH
0445 8409            JMP   GNPTS


0447 B920  ANLAST    MOV   R1, NoO2OH   ;NOT LAST PTS, GET SAMPLE
0449 F1              MOV   A, CdR1
044A 9453            CALL  SSCALE
044C 60              ADD   A, CdRO      ;ADD TO LS BYTE
044D AO              MOV   CdRO, A
044E E652            JNC   AVDONE
0450 18              INC   RO
0451 10              INC   CdRO         ;ADD CY TO MS BYTE
0452 83    AVDONE    RET


            SCALING ROUTINE
           SHIFTS A BASED ON BITS 0,1 OF R4


0453 FC    SSCALE    MOV   A, R4        ;A < - SCALE PARAM
0454 5279            JB2   ACSCALE      ;BIT2=1 => SCALE ABOUT 128
0456 125C            JBO   S1OR3        ;TEST WHICH SCALE FACTOR
0458 326E            JB1   S2
045A F1              MOV   A, CdR1      ;SCALE=0 => NO SCALE
045B 83              RET
045C 2363  S1OR3     JB1   S3
045E F1              MOV   A, CdR1      ;SCALE=1 => *2
045F E7              RL    A
0460 1276            JBO   SOVRFLO
0462 83              RET
0463 F1    S3        MOV   A, CdR1      ;SCALE=3 => *8
0464 E7              RL    A
0465 1276            JBO   SOVRFLO
0467 E7              RL    A
0468 1276            JBO   SOVRFLO
046A E7              RL    A
046B 1276            JBO   SOVRFLO
```

```
046D 83                  RET
046E F1      S2          MOV   A, CdR1      ;SCALE=2 => *4
046F E7                  RL    A
0470 1276                JB0   SOVRFLO
0472 E7                  RL    A
0473 1276                JB0   SOVRFLO
0475 83                  RET
0476 23FF    SOVRFLO     MOV   A, NoOFFH
0478 83                  RET


0479 F1      ACSCALE     MOV   A, CdR1      ;A < - SAMPLE
047A F2BB                JB7   ACPOS        ;TEST IF POSITIVE OR
                                             NEGATIVE


047C FC                  MOV   A, R4        ;A < - SCALE PARAM
047D 1283                JB0   AS1OR3 .     ;TEST WHICH SCALE FACTOR
047F 32A9                JB1   AS2
0481
0481 F1                  MOV   A, CdR1      ;NO SCALE FACTOR HERE
0482 83                  RET


0483 3294    AS1OR3      JB1   AS3

0485 F1                  MOV   A, CdR1      ;SCALE PARAM IS 1
0486 37                  CPL   A            ;TAKE ABSOLUTE VALUE
0487 537F                ANL   A, NoO7FH
0489 17                  INC   A
048A F2E8                JB7   UNDRFLO
048C E7                  RL    A            ;PERFORM SCALE
048D F2E8                JB7   UNDRFLO
048F 37                  CPL   A
0490 537F                ANL   A, NoO7FH
0492 17                  INC   A
0493 83                  RET


0494 F1      AS3         MOV   A, CdR1      ;SCALE PARAM IS 3
0495 37                  CPL   A            ;TAKE ABSOLUTE VALUE
0496 537F                ANL   A, NoO7FH
0498 17                  INC   A
```

```
0499 F2E8              JB7   UNDRFLO
049B E7               RL    A           ;PERFORM SCALE
049C F2E8              JB7   UNDRFLO
049E E7               RL    A
049F F2E8             JB7   UNDRFLO
04A1 E7               RL    A
04A2 F2E8             JB7   UNDRFLO
04A4 37               CPL   A
04A5 537F             ANL   A, No07FH
04A7 17               INC   A
04A8 83               RET


04A9 F1        AS2    MOV   A, CdR1     ;SCALE PARAM IS 2
04AA 37               CPL   A           ;TAKE ABSOLUTE VALUE
04AB 537F             ANL   A, No07FH
04AD 17               INC   A
04AE F2E8             JB7   UNDRFLO
04B0 E7               RL    A           ;PERFORM SCALE
04B1 F2E8             JB7   UNDRFLO
04B3 E7               RL    A
04B4 F2E8             JB7   UNDRFLO
04B6 37               CPL   A
04B7 537F             ANL   A, No07FH
04B9 17               INC   A
04BA 83               RET


04BB FC        ACPOS  MOV   A, R4       ;A < - SCALE PARAM
04BC 12C2             JB0   PS1OR3      ;TEST WHICH SCALE FACTOR
04BE 32DC             JB1   PS2

04C0 F1               MOV   A, CdR1     ;SCALE PARAM = 0
04C1 83               RET


04C2 32CD      PS1OR3 JB1   PS3

04C4 F1               MOV   A, CdR1     ;SCALE PARAM = 1
04C5 537F             ANL   A, No07FH   ;TAKE ABSOLUTE VALUE
04C7 E7               RL    A           ;PERFORM SCALE
04C8 F276             JB7   SOVRFLO
```

```
04CA 4380              ORL   A, No080H
04CC 83                RET
04CD F1      PS3       MOV   A, CdR1      ;SCALE FACTOR = 3
04CE 537F              ANL   A, No07FH    ;TAKE ABSOLUTE VALUE
04D0 E7                RL    A            ;PERFORM SCALE
04D1 F276              JB7   SOVRFLO
04D3 E7                RL    A
04D4 F276              JB7   SOVRFLO
04D6 E7                RL    A
04D7 F276              JB7   SOVRFLO
04D9 4380              ORL   A, No080H
04DB 83                RET


04DC F1      PS2       MOV   A, CdR1      ;SCALE FACTOR = 2
04DD 537F              ANL   A, No07FH    ;TAKE ABSOLUTE VALUE
04DF E7                RL    A            ;PERFORM SCALE
04E0 F276              JB7   SOVRFLO
04E2 E7                RL    A
04E3 F276              JB7   SOVRFLO
04E5 4380              ORL   A, No080H
04E7 83                RET


04E8 2300   UNDRFLO    MOV   A, NoOH
04EA 83                RET
```

## TIMER INTERRUPT ROUTINE

```
04EB 65     TIMERI     STOP  TCNT         ;RELOAD TIMER AND USE UP
04EC AF                MOV   R7, A        ;SAVE ACCUMULATOR
04ED 23F5              MOV   A, No         ;20 MICROSECS
                             (OFFH-OAH)    (ABOUT 14 CYCLES)
04EF 62                MOV   T, A
04F0 FF                MOV   A, R7        ;RESTORE ACCUMULATOR
04F1 00                NOP
04F2 25                EN    TCNTI
04F3 55                STRT  T
04F4 93                RETR
```

```
06F0                ORG    06F0H        ;LAST HOPE IF CRASH
06F0 E479           JMP    SRESET
0000                END
```

0235312

## SYMBOL TABLE

```
ACPOS    04BB        CODE10  00B5    IAVGAD  0400      NTDOWN  03
3E          STL1     03E5
ACSCALE  0479        CODE2   00A5    ICODE0  00B7      NZ      00
0C          TCMD     0744
ADSC     0235        CODE3   00A7    ICODE1  00B9      OVDONE  02
C8          TD7      07E2
ANLAST   0447        CODE4   00A9    ICODE10 00CB      OVERFLO 02
F7          TEVT     034B
AOVRFLO  0443        CODE5   00AB    ICODE2  00BB      OVFLO   02
DD          TIMERF   006F
AS1OR3   0483        CODE6   00AD    ICODE3  00BD      OVRFLO  02
F6          TIMERI   04EB
AS2      04A9        CODE7   00AF    ICODE4  00BF      PEAKM   01
25          TPMO     07CD
AS3      0494        CODE8   00B1    ICODE5  00C1      PEAKP   01
09          TRIG     03B1
ASHIFTS  0435        CODE9   00B3    ICODE6  00C3      PMDONE  01
37          TRTRY1   03C0
ATN      0700        DDONE   015C    ICODE7  00C5      POS     02
1C          TRTRY2   03CF
ATOD     00EE        DIFF    0146    ICODE8  00C7      PPDONE  01
1B          TRTRY3   03E6
ATRY1    0411        DOAVG   069F    ICODE9  00C9      PROCESS 00
7F          TRY100   0177
ATRY2    0416        DONE    07EB    IDIFF   0138      PS1OR3  04
C2          TRY150   0181
ATRY3    041B        DPOVFLO 0371    IEMG    0169      PS2     04
DC          TRY200   018B
AVCH1    07B7        DPROTL  02E0    IEVTT   02FB      PS3     04
CD          TRYB3    0774
AVDONE   0452        DPROTL2 02E8    IHALT   00D4      PUTSAMP 07
6D          TRYTROU  0154
AVERG    0680        EDUMP2  0226    IHD     00E3      RECTIFY 02
09          TSF      0256
AVGAD    0421        EIEMG   0199    INIT    0009      RESET   00
CD          TSF10    02BF
B40      0717        EMG     0200    IPEAKM  011C      S1OR3   04
5C          TSF2     025F
```

| Symbol | Addr | Symbol | Addr | Symbol | Addr | Symbol | Addr | Symbol | Addr |
|---|---|---|---|---|---|---|---|---|---|
| B416E | 0735 | TSF3 | 0268 | EMGDONE | 02C7 | IPEAKP | 0100 | S2 | 04 |
| B4B563 | 06BE | TSF4 | 0275 | ENOINC1 | 0214 | ITEVT | 033F | S3 | 04 |
| B4N5A3 | 06C6 | TSF5 | 027E | ENOINC2 | 0225 | ITRIG | 03A6 | SAPL1 | 07 |
| B5B9 | 06AB | TSF6 | 028B | EOCO | 004F | JMPTBL1 | 0087 | SAPL2 | 07 |
| B5095 | 071D | TSF7 | 0298 | EOC1 | 0068 | JMPTBL2 | 0096 | SETAPT | 07 |
| B51E0 | 073B | TSF8 | 02A9 | EQ1 | 022F | LOCP1 | 0750 | SETD6 | 07 |
| B5B40E9 | 0721 | TSF9 | 02B2 | ETNEQ | 0313 | M34 | 076B | SETD7 | 07 |
| B5B41D5 | 073F | TTHRESH | 031F | EVTT | 030A | MIDNIB | 02CD | SETHR | 07 |
| BODY287 | 0544 | TTRY1 | 035B | FIRST | 0092 | NBOR | 0690 | SETPS | 07 |
| BODYD8B | 054D | TTRY2 | 0385 | GE2 | 0233 | NCAR | 0699 | SETSC | 07 |
| BODYLG76 | 053D | UNDRFLO | 04E8 | GNPTS | 0409 | NCBITS | 0776 | SOVRFLO | 04 |
| CBITS79 | 07D9 | WAIT | 0073 | GSIZE0 | 0723 | NEEDO | 0330 | SRESET | 07 |
| CHNLO53 | 003D | YINT | 0703 | GSIZE1 | 0741 | NOEOC | 074E | SSCALE | 04 |
| CHNL1A5 | 0054 | ZERO | 0000 | HALT | 00E4 | NOF | 03F8 | STILO | 03 |
| CMD84 | 075A | ZSUM | 0193 | HD2 | 00EB | NOT256 | 0376 | STIL1 | 03 |
| CN15A | 0728 | | | HERE | 007A | NOTEVT | 032F | STILLO | 03 |
| CODEOCE | 00A1 | | | HYSTER | 03EC | NOWAIT | 007C | STILLLO | 03 |
| CODE1BF | 00A3 | | | IADONE | 0420 | NT256 | 0396 | STILLL1 | 03 |

**Patent Claims:**

1. A training device for selectively monitoring one or more of a plurality of physiological signals produced by a human being and for displaying computed measures of such signals including:

   (a) a plurality of replaceable, selectable and interchangeable transducer means adapted to be singly and plurally utilized for conversion of said plurality of equivalent electrical signals;

   (b) a plurality of corresponding signal conditioning means for said equivalent electrical signals including adjustable electrical amplification means, filtering means and conversion means adapted to convert said plurality of said conditioned equivalent electrical signals into a corresponding plurality of equivalent equipotential pulse trains, each of whose instantaneous frequencies is proportional to each of said corresponding plurality of said conditioned equivalent electrical signals;

   (c) a plurality of display means operative for presentation of said plurality of equivalent equipotential pulse train as computed measures of said physiological signals;

   (d) terminal means for control of said training device by the said human being;

   (e) a plurality of relatively independent computer means cooperating in selective combinations and at least one combination of which is adapted to calculate said computed measures of said corresponding plurality of equivalent equipotential pulse trains, and another combination of which is adapted to control at least one of said plurality of display means;

   (f) supervising computer means adapted to control, select and/or coordinate said plurality of said relatively independent computer means; and

2    0235312

(g) communication bus means adapted to transmit information between said plurality of relatively independent
computer means and said supervising computer means.

2. A training device as in claim 1 wherein said plurality of
display means each includes at least one of:
(a) visual display means for the presentation of visual
stimuli or pictures, and
(b) auditory display means for the presentation of audible
stimuli or sounds to said human being.

3. A training device as in claim 1 wherein said plurality of
replaceable, selectable and interchangeable transducer
means each includes at least one of:
(a) electrode means adapted to conduct electrical activity
produced by said human being to said device;
(b) photo detection means and a lilght source in optical
proximity to said photo detection means wherein light
from said light source is modulated by physiological
signals such as blood flow before reaching said photo
detection means wherein electrical activity is
produced in correspondence with said modulation and
coupled to said device;
(c) variable resistance means such that variation of human
limb or body volume or position causes corresponding
variation in resistance of said resistance means which
is coupled to said device;
(d) piezoelectric means adapted to generate electrical
change in correspondence with variation or force or
pressure upon said piezoelectric means by physiological signals such as activity of human muscle which is
coupled to said device.

4. A training device as in claim 1 wherein:
(a) a plurality of transmitters are provided corresponding
with said plurality of replaceable, selectable and
interchangeable transducer means, said plurality of
replaceable, selectable and interchangeable transducer means being adapted to modulate a corresponding
plurality of electrical carrier signals with information equivalent to said physiological signals;

(b) wherein a plurality of corresponding remote receiver
means are provided to which said information is
transmitted; and

(c) wherein said information is demodulated and coupled to
said signal conditioning means.


5. A training device as in claim 1 wherein each of said
plurality of signal conditioning means further comprises:

(a) preamplifier means adapted to amplify electrical
signals received from any one or more of said plurality of replaceable, selectable and interchangeable
transducer means such that physiological information
is contained in the instantaneous variation of the
magnitude of said electrical signals;

(b) adjustable gain amplifier and filter means including
adjustable electrical amplification factor means
adapted to further amplify electrical signals received
from said preamplifier means;

(c) gain switch means adapted to vary the said adjustable
electrical amplification factor of said adjustable
gain amplifier and filter means;

(d) voltage to frequency converter means adapted to
receive electrical signals from said adjustable gain
amplifier and filter means and to generate an equipotential pulse train wherein the instantaneous
frequency of said pulse train is in correspondence to
the instantaneous variation of the magnitude of said
electrical signals;

(e) photo detector means and opto-isolator means including
a first light source which is in close proximity
thereto, said opto-isolator means being adapted to
convert said equipotential pulse train into a corresponding and second equipotential pulse train; and

(f) a second and plurality of photo detector means and a
plurality of second opto-isolator means including a
second plurality of light source means and in close proximity to said second plurality of photo detector means, said second equipotential pulse train being transmitted to at least one of said plurality of relativey independent computer means and being adapted to receive pulses from said plurality of relatively independent computers and to transit said pulses to said gain switch.

6. A training device as in claim 1 wherein each of said plurality of relatively independent computer means further comprises:

(a) central processing means wherein arithmetic operations, comparison of numbers, logical operations, and interpretation and execution of instructions are performed;

(b) program memory means containing a plurality of binary sequences adapted to contain control sequences from said central processing means;

(c) data memory means adapted to contain a second plurality of binary sequences inlcuding: results of arithmetic and logical operations performed in said central processing means, a summation of said equipotential pulse trains from said signal conditioning means, and parameter sequences from said supervising computer means;

(d) a plurality of timer means adapted to accept a third plurality of binary sequences from said central processing means and to produce a fourth binary sequence after a time determined by said third plurality of binary sequences;

(e) a plurality of parallel input/output means adapted to connect each of said plurality of relatively independent computer means to said communication bus means, and additionally adapted to couple each of said plurality of display means; and

(f) local bus means adapted to transit information between said plurality of parallel input/output means, said central processing means, said program memory means, said data memory means, and said plurality of timer means.

7. A training device as in claim 6 wherein the first mentioned of said plurality of binary sequences in said program memory means further comprises:

(a) integration binary sequence means adapted to control said central processing means, said data memory means, said plurality of timer means, and said plurality of parallel input/output means such that the number of said equipotential pulses per unit of time can be transmitted to said plurality of parallel input/output means and to said data memory means, such unit of time being controlled by said fourth binary sequence;

(b) peak binary sequence means adapted to control said central processing means, said data memory means, said plurality of timer means, and said plurality of parallel input/output means such that the maximum frequency of said equipotential pulse train in a unit of time can be transmitted to said plurality of parallel input/output means and to said data memory means, such unit of time being controlled by said fourth binary sequence;

(c) peak to peak binary sequence means adapted to control said central processing means, said data memory means, said plurality of timer means, and said plurality of parallel input/output means such that the difference between the maximum frequency and the minimum frequency of said equipotential pulse train in a unit of time can be transmitted to said plurality of parallel input/output means and to said data memory means, such unit of time being controlled by said fourth binary sequence;

(d) analog conversion sequence means adapted to control said central processing means, said data memory means, said plurality of timer means, and said plurality of parallel input/output means such that the frequency of said equipotential pulse train can be transmitted to said plurality of parallel input/output means and to said data memory means, such transmission being determined at the time controlled by said fourth binary sequence;

(e) normalizing binary sequence means adapted to control said central processing means, said data memory means, said plurality of timer means, and said plurality of parallel input/output means such that said gain switch means is adapted to sequence said electrical amplification factor of said adjustable gain amplifier and filter means from highest value to such value as produces a predetermined frequency of said equipotential pulse train, such predetermined frequency being proportional to the maximum allowed voltage which can be generated by said adjustable gain amplifier and filter means which is consistent with said replaceable, selectable and interchangeable (physiological) transducer means;

(f) binary visual display sequence means adapted to control said central processing means, said data memory means, said plurality of timer means, and said plurality of parallel input/output means such that a visual presentation or picture appears on said visual display means; and

(g) binary auditory display sequence means adapted to control said central processing means, said data memory means, said plurality of timer means, and said plurality of parallel input/output means such that an audible presentation, stimulus or sound issues from said auditory display means.

8. A training device as in claim 1 wherein said supervising computer means further comprises:

(a) second central processing means wherein arithmetic operations, comparison of numbers, logical operations, and interpretation and execution of instructions are performed;

(b) interchangeable program memory means containing a fifth plurality of binary sequences adapted to control said second central processing means, said interchangeable program memory means being capable of being removed from said supervising computer means and replaced with at least one of plurality of a second interchangeable program memory means containing a sixth plurality of binary sequences adapted to control said second central processing means;

(c) second data memory means adapted to contain a seventh plurality of binary sequences including results of arithmetic and logical operations performed in said second mentioned central processing means, results of computations executed in each of said plurality of computer means, and information transmitted from said terminal means;

(d) second plurality of timer means adapted to receive an eighth plurality of binary sequences from said second central processing means and to generate a ninth binary sequence after a period of time determined by said eighth plurality of binary sequence;

(e) communication port means adapted to receive information from said terminal means and to transmit information to said terminal means;

(f) communcation bus central means adapted to receive information from said communication bus means and to transmit information to said communication bus means; and

(g) second local bus means adapted to transmit information between said second central processing means, said second data memory means, said second plurality of timer means, said communication port means and said communication bus central means.

9. A training device as in claim 8 wherein said interchangeable program memory means contains at least one of a tenth plurality of binary sequences and:

(a) adapted to control said second central processing means, said second data memory means, said second plurality of timer means, said communication port means and said communication bus control means such that an eleventh plurality of binary sequences is transmitted to said plurality of relatively independent computer means creating at least one of a plurality of visual or audiotory presentations on at least one of the said plurality of display means, or

(b) adapted to control said second central processing means, said second data memory means, said second plurality of timer means, said communication port means, and said bus control means such that said information transmitted from said plurality of relatively independent computer means can be compared to a selected or computed range of values and such that an error signal proportional to the difference between said information and said selected or computed range is calculated, such error being retransmitted to said plurality of relatively independent computer means, wherein it modifies or alters at least one of said plurality of display means.

10. A method of improving skilled behavior or performance by and for a human being comprising the steps of:

(a) receiving his or her plurality of signals as physiological information at successive instants of time regarding behavior or performance from one or more of a plurality of replaceable, selectable and interchangeable transducer means;

(b) quantifying said physiological information underlying such behavior or performance of said successive instants of time by means of at least one of a plurality of relatively independent and cooperating computer means each having a plurality of discrete functions and adapted to perform at least one of a plurality of discrete mathematical calculations of said successive instants of time and whose results provide a measure of at least one specific aspect of physiological behavior or performance at each of said successive instants of time;

(c) comparing said measured physiological behavioral information at each of said successive instants of time against a predetermined measure for said each of successive instants of time considered to be optimal for said one specific aspect of physiological behavior or performance and calculating by means of a supervising computer means an error quantity for said each of successive instants of time, such error quantity being the difference between the said predetermined measure and said measure of said one specific aspect of physiological behavior or performance for said each of successive instants of time; and

(d) displaying by means of at least one of a plurality of selective display means either the said predetermined measure (,) considered to be optimal of said one specific aspect of physiological behavior or performance, or the said error quantity, said one of a plurality of selective display means showing or reflecting at least one of said predetermined measures of said specific one aspect of physiological behavior or performance of said error quantity, said plurality of selective display means being controlled by at least one of said relatively independent and cooperating computer means, said relatively independent and cooperating computer means being adapted to modify any one of said plurality of selective display means.

11. The method defined in claim 10 further including:
   (a) utilization of said plurality of selective display means by a user said human being and affording an opportunity for improvement of skilled performance or behavior;

(b) including the steps of progressive modification of performance or behavior by him or her with subsequent reduction of said error quantity.

12. The method defined in claim 10 wherein step (a) thereof includes at least one of the steps of receiving information regarding the position of limbs or other parts of the human body, or receiving a plurality of myographic, encephalic, or cardiographic or hemodynamic informtion either singly or in any combination thereof, and wherein: step (b) thereof includes at least one of the steps of calculating the integral of such behavior or performance as a function of time, or the calculation of peak magnitude or variation of such behavior or performance per unit of time as a function of time, or the calculation of the excursion or peak to peak variation of such behavior or performance per unit of time as a function of time, or the calculation of the instantaneous variation or magnitude of such behavior or performance as a function of time, and wherein: step (c) thereof further includes the step of calculating a second error which assumes one of two values, taking a first value when the absolute value of the difference between the said calculated measure and the said predetermined measure is in excess of a predetermined lower bound and taking a second value when the absolute value of the difference between the said calculated measure and the said predetermined measure is smaller than said predetermined lower bound, said predetermined lower bound being a predetermined function of both the absolute value of the difference between the said calculated measure and the said predetermined measure and time, and wherein: step (d) thereof includes at least one of the steps of representation of such information on a television receiver or the like, such representation being in the form of a picture familiar to human beings such that variables associated with said picture are proportional to either said calculated measure of behavior or performance, or said first mentioned error or said second error either individually or in combination, or representation
of such information on a speaker or the like, such
representation being in the form of sounds familiar to
human beings such that variables associated with such
sounds are proportional to either the said calcuiated
measure of such behavior or performance, said first
mentioned error or said second error, or the said
predetermined measure of such behavior or performance
either individually or in combination.

13. A method of improving physical fitness or well-being of a
human being comprising the steps of:
(a) receiving his or her physiological information
regarding behavior or performance or well-being from
a plurality of replaceable, selectable and interchangeable transducers;
(b) quantifying said physiological information underlying
such behavior or performance or well-being by means
of at least one of a plurality of relatively independent cooperating computers adapted to perform at
least one of a plurality of discrete mathematical
calculations whose results provide a measure of at
least one specific aspect of physiological behavior
or performance or well-being;
(c) quantifying a predetermined measure based upon said
received and quantified physiological information
considered to be optimal as a function of said
physiological information and time for the specific
aspect of physiological behavior or performance or
well-being representative of the source of such
physiological information by means of supervising
computer means;
(d) receiving his or her successive physiological
information regarding their respective continued
state of behavior or performance or well-being as a
function of time from said plurality of replaceable,
selectable and interchangeable transducers;

(e) quantifying said successive physiological information underlying such behavior or performance or well-being;

(f) comparing the quantified information from said successive physiological information against said predetermined measure considered to be optimal and calculating by means of at least one of said plurality of relatively independent cooperating computers an error quantity, such error quantity being the difference between said predetermined measure and said quantified measure of said successive physiological information at each successive instant of time, and

(g) displaying either said predetermined measure considered to be optimal, said quantified measure of said successive physiological information, or said calculated error quantity by means of at least one selective display means, as a function of time either individually or in any combination.

14. The method defined in claim 13 wherein:

step (a) and (b) thereof include at least one of the steps of receiving at least one of a plurality of galvanic, myographic, encephalic, cardiographic and hemodynamic information from said human being, and wherein

steps (d) and (e) thereof include at least one of the steps of calculation of the integral of said physiological information or said successive physiological information, or calculation of the peak magnitude or variation of said physiological information or said successive physiological information, per unit of time as a function of time, or calculation of the excursion or peak to peak variation of said physiological information or said successive physiological information, per unit of time as a function of time, or calculation of the excursion or peak to peak variation of said physiological information or said successive physiological information

per unit of time as a function of time by means of at least one of said plurality of relatively independent cooperating computer means; and step (f) thereof includes at least one of the steps of calculation of an error quantity consisting of the difference between said successive physiological information and said predetermined measure considered to be optimal, or calculation of an error quantity which assumes one of two values, a first value when the absolute value of the difference between said successive physiological information and said predetermined measured considered to be optimal exceeds a predetermined value, and a second value when said absolute value of said difference does not exceed said predetermined value by means of said supervising computer means, and wherein: step (g) thereof includes the step of representation of at least one selective display means on a television receiver or the like being in the form of a picture familiar to human beings such that variables associated with said picture are proportional to either said successive physiological information, said predetermined value considered to be optimal, or said error, or representation of said one selective display means on speaker or the like, being in the form of sounds familiar to human beings such that variables associated with said sounds are proportional to either said successive physiological information, said predetermined value considered to be optimal, or said error by means of at least one of said relatively independent cooperating computer means.

15. A system for selectively monitoring one or more physiological reactions generated by an individual and for generating signal representations of the monitored reactions said signals having characteristics which correspond to the characteristics of said monitored reactions and for displaying said signal representations including:

(a) sensor means coupled to said individual for monitoring a pre-selected body part of said individual and for generating electric signals representing said pre-selected body part, said electric signals having characteristics which correspond to characteristics of the reactions of said pre-selected body part;

(b) means for applying said electric signals to a pre-processor means for processing said electric signals into data signals and for applying said data signals to a host computer, said pre-processor means including a central processing unit programmed to request and receive said electric signals in a predetermined form and for performing calculations on said signals of predetermined form for making data signals, conversion means for receiving said request and for receiving said electric signals from said sensor means and for converting said electric signals into said predetermined form and for sending said signals in said predetermined form to said central processing unit and interface means for coupling said central processing unit to input/output lines of a host computer;

(c) said pre-processor means coupled to said host computer via said computer input/output lines through said interface means, for receiving data signals from said central processing unit of said pre-processor means; and

(d) monitor means coupled to said host computer for displaying said data signals received by said central processing unit for displaying representations of said monitored reactions.

# FIG.8

# FIG.1

FIG. 2

*System Block Diagram*

FIG.3

FIG. 3a

# FIG.4

80 — START

81 — INITIALIZATION

82 — START CLOCK

83 — GET CHANNEL 3 DATA

84 — SET ADC TO PROCESS CHANNEL 2 SIGNAL

85 — A

95 — RETURN

101 — B

YES

REQUEST FROM HOST? — 100

NO

96 — RETURN

GET CHANNEL 2 DATA — 83a

SET ADC TO PROCESS CHANNEL 1 SIGNAL — 84a

85a — A

101a — B

YES

REQUEST FROM HOST? — 100a

NO

96b — 

CLOCK INTERRUPT?   YES

NO

0235312

# FIG. 5

85 — START A

86 — WAS PARA-METER OR COMMAND JUST CHANGED ?

87 — YES     NO — 88

COMMAND = 0 ? —YES→ SOFTWARE RESET IF PROCESSOR ←YES— COMMAND = 0 ?

RETURN — 95

DATA = 16

COMMAND = 0 ? → NO

COMMAND = 1 ? —YES→ TIME PARAMTETER = 61? ←YES— COMMAND = 1 ?

TIME PARAMTETER = 61? → NO

OUT MODULE DATA

RETURN — 95

RETURN — 95

COMMAND = 1 ? → NO

COMMAND = 2? —YES→ DATA 3 SCALE A/D ←YES— COMMAND = 2?

RETURN — 95

COMMAND = 2? → NO

C — 90

D

91 — COMMAND = 2? → NO

FIG. 5a

# FIG. 5b

```
                    101 ─╮   ┌─────────┐
                         ╰──( START B  )
                             └─────────┘
                                  │
                                  ▼
                          ┌────────────────┐
                          │ TEST WHICH     │
                          │ CHANNEL        │
                          └────────────────┘
                                  │
                                  ▼
          GET                ╱ NEW           ╲            NEW
        ───────────────◄   ╱  COMMAND OR GET  ╲  ──────────────────►
          DATA            ╲   DATA ?          ╱         COMMAND
           │               ╲                 ╱                │
           ▼                 ╲             ╱                  ▼
  ┌────────────────┐                            PARAMETER  ╱ CHANGE      ╲  COMMAND
  │ COMPUTE        │                          ──────────◄ ╱ COMMAND OR    ╲ ─────────►
  │ SMOOTHIER      │                            │        ╲ PARAMETER ?    ╱    │
  │ AVERAGE        │                            │         ╲              ╱     │
  └────────────────┘                            ▼                              ▼
           │                         ┌──────────────────┐         ┌──────────────────┐
           ▼                         │ GET PARAMETER    │         │ GET COMMAND      │
  ┌────────────────┐                 │ VALUE FROM HOST  │         │ FROM HOST        │
  │ OUT PUT DATA   │                 └──────────────────┘         └──────────────────┘
  │ TO HOST        │                          │                            │
  └────────────────┘                          ▼                            ▼
           │                         ┌──────────────────┐         ┌──────────────────┐
           ▼                         │ STORE            │         │ STORE            │
      ( RETURN )                     │ PARAMETER        │         │ COMMAND          │
           │                         │ VALUE            │         └──────────────────┘
          96                         └──────────────────┘                  │
                                              │                            ▼
                                              ▼                       ( RETURN )
                                         ( RETURN )                        │
                                              │                           96
                                             96
```

FIG.6

FIG.7

FIG.9

0235312

**FIG. 10**

FIG. 11

FIG. 11a